(19) Europäisches Patentamt
European Patent Office
Office européen des brevets

(11) **EP 2 731 715 B1**

(12) **EUROPÄISCHE PATENTSCHRIFT**

(45) Veröffentlichungstag und Bekanntmachung des Hinweises auf die Patenterteilung:
**11.12.2019   Patentblatt 2019/50**

(51) Int Cl.:
*B01J 23/882* (2006.01)   *C07C 5/00* (2006.01)
*B01J 35/02* (2006.01)   *B01J 35/10* (2006.01)
*B01J 37/02* (2006.01)   *B01J 37/04* (2006.01)
*B01J 37/08* (2006.01)   *B01J 35/00* (2006.01)
*B01J 23/887* (2006.01)   *C01G 39/00* (2006.01)

(21) Anmeldenummer: **12733719.4**

(22) Anmeldetag: **11.07.2012**

(86) Internationale Anmeldenummer:
**PCT/EP2012/063530**

(87) Internationale Veröffentlichungsnummer:
**WO 2013/007736 (17.01.2013 Gazette 2013/03)**

(54) **MO, BI UND FE ENTHALTENDE MULTIMETALLOXIDMASSEN**

MULTI-METAL OXIDE MASSES CONTAINING MO, BI AND FE

MASSES D'OXYDE MÉTALLIQUE CONTENANT DU MO, DU BI ET DU FE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.07.2011   DE 102011079035**
**12.07.2011   US 201161506693 P**
**05.10.2011   DE 102011084040**
**05.10.2011   US 201161543333 P**

(43) Veröffentlichungstag der Anmeldung:
**21.05.2014   Patentblatt 2014/21**

(73) Patentinhaber: **BASF SE**
**67056 Ludwigshafen am Rhein (DE)**

(72) Erfinder:
• **MACHT, Josef**
**68159 Mannheim (DE)**
• **KARPOV, Andrey**
**Metuchen, NJ 08840 (US)**
• **DOBNER, Cornelia Katharina**
**67071 Ludwigshafen (DE)**
• **ROSOWSKI, Frank**
**68239 Berlin (DE)**
• **HAMMON, Ulrich**
**68163 Mannheim (DE)**
• **MÜLLER-ENGEL, Klaus Joachim**
**76297 Stutensee (DE)**

(74) Vertreter: **BASF IP Association**
**BASF SE**
**G-FLP-C006**
**67056 Ludwigshafen (DE)**

(56) Entgegenhaltungen:
**DE-A1-102007 003 076     US-A1- 2011 077 148**

Bemerkungen:
Die Akte enthält technische Angaben, die nach dem Eingang der Anmeldung eingereicht wurden und die nicht in dieser Patentschrift enthalten sind.

**Beschreibung**

Vorliegende Erfindung betrifft Mo, Bi und Fe enthaltende Multimetalloxidmassen der allgemeinen Stöchiometrie I,

$$Mo_{12}Bi_aCo_bFe_cK_dSi_eO_x \qquad (I),$$

in der die Variablen folgende Bedeutung haben:

a = 0,5 bis 1,
b = 7 bis 8,5,
c = 1,5 bis 3,0,
d = 0 bis 0,15,
e = 0 bis 2,5 und
x = eine Zahl, die durch die Wertigkeit und Häufigkeit der von Sauerstoff verschiedenen Elemente in I bestimmt wird, und die folgenden Bedingungen erfüllen:

Bedingung 1: 12 - b - 1,5 • c = A,
und
$0,5 \leq A \leq 1,5$;

Bedingung 2: $0,2 \leq a/A \leq 1,3$; und

Bedingung 3: $2,5 \leq b/c \leq 9$.

Außerdem betrifft vorliegende Erfindung Verfahren zur Herstellung von Multimetalloxidmassen der allgemeinen Stöchiometrie I, sowie deren Verwendung als katalytische Aktivmassen von Katalysatoren für die heterogen katalysierte partielle Gasphasenoxidation organischer Verbindungen, insbesondere derjenigen von Propen zu Acrolein als Hauptprodukt sowie Acrylsäure als Nebenprodukt.

Mo, Bi und Fe enthaltende Multimetalloxidmassen der allgemeinen Stöchiometrie I, die die Bedingungen 1, 2 und 3 nicht erfüllen, sind z.B. aus der DE-A 19855913 bekannt.

Aus der DE-A 19855913 ist auch bekannt, solche Multimetalloxidmassen als Aktivmassen von Katalysatoren für die heterogen katalysierte partielle Gasphasenoxidation von Propen zu Acrolein als Hauptprodukt und Acrylsäure als erwünschtem Nebenprodukt einzusetzen (Acrylsäure ist insbesondere deshalb erwünschtes Nebenprodukt, weil die heterogen katalysierte partielle Gasphasenoxidation von Propen zu Acrolein vor allem als erste Oxidationsstufe der zweistufigen Partialoxidation von Propen zu Acrylsäure Anwendung findet).

Nachteilig an den Multimetalloxidmassen der DE-A 19855913 als Aktivmassen von Katalysatoren für die heterogen katalysierte partielle Gasphasenoxidation von Propen zu Acrolein als Hauptprodukt und Acrylsäure als erwünschtem Nebenprodukt ist jedoch insbesondere, dass die resultierende Gesamtselektivität der Bildung von Acrolein und Acrylsäure nicht in vollem Umfand zu befriedigen vermag.

Das bezüglich der Multimetalloxidmassen der DE-A 19855913 Gesagte trifft auch auf die Mo, Bi und Fe enthaltenden Multimetalloxidmassen der DE-A 10063162, der DE-A 102005037678, der DE-A 10059713, der DE-A 10049873, der DE-A 102007003076, der DE-A 102008054586, der DE-A 102007005606 und der DE-A 102007004961 zu.

Die Aufgabe der vorliegenden Erfindung bestand daher insbesondere darin, Mo, Bi und Fe enthaltende Multimetalloxidmassen zur Verfügung zu stellen, die als Aktivmassen von Katalysatoren für die heterogen katalysierte partielle Gasphasenoxidation von Propen zu Acrolein als Hauptprodukt und Acrylsäure als Nebenprodukt eine verbesserte Gesamtselektivität der Bildung von Acrolein und Acrylsäure (d.h., eine verbesserte Wertproduktgesamtselektivität) ermöglichen.

Als Lösung der Aufgabe werden Mo, Bi und Fe enthaltende Multimetalloxidmassen der allgemeinen Stöchiometrie I,

$$Mo_{12}Bi_aCo_bFe_cK_dSi_eO_x \qquad (I),$$

in der die Variablen folgende Bedeutung haben:

a = 0,5 bis 1,
b = 7 bis 8,5,

c = 1,5 bis 3,0,
d = 0 bis 0,15,
e = 0 bis 2,5 und
x = eine Zahl, die durch die Wertigkeit und Häufigkeit der von Sauerstoff verschiedenen Elemente in I bestimmt wird, und die folgenden Bedingungen erfüllen:

Bedingung 1: $12 - b - 1{,}5 \cdot c = A$,
und
$0{,}5 \leq A \leq 1{,}5$;

Bedingung 2: $0{,}2 \leq a/A \leq 1{,}3$; und

Bedingung 3: $2{,}5 \leq b/c \leq 9$,

zur Verfügung gestellt.

Erfindungsgemäß bevorzugt ist der stöchiometrische Koeffizient d 0,04 bis 0,1, und besonders bevorzugt 0,05 bis 0,08.

Der stöchiometrische Koeffizient e beträgt erfindungsgemäß vorteilhaft 0,5 bis 2, und besonders vorteilhaft 0,8 bis 1,8 bzw. 1 bis 1,6.

Darüber hinaus gilt für die Bedingung 1 vorteilhaft $0{,}5 \leq A \leq 1{,}25$, und besonders vorteilhaft $0{,}5 \leq A \leq 1$.

Für die Bedingung 2 gilt erfindungsgemäß bevorzugt $0{,}3 \leq a/A \leq 1{,}2$, und besonders bevorzugt $0{,}4 \leq a/A \leq 1{,}2$, sowie ganz besonders bevorzugt $0{,}5 \leq a/A \leq 1{,}2$.

Der Quotient b:c = b/c (die Bedingung 3) erfüllt erfindungsgemäß vorteilhaft die Relation $3 \leq b/c \leq 9$, besonders vorteilhaft die Relation $3 \leq b/c \leq 7$, und ganz besonders vorteilhaft die Relation $3 \leq b/c \leq 5$.

Ganz besonders bevorzugte Multimetalloxidmassen der allgemeinen Stöchiometrie I sind somit jene, bei denen gleichzeitig gilt:

d = 0,04 bis 0,1;
e = 0,5 bis 2;

$$0{,}5 \leq A \leq 1{,}25;$$

$$0{,}4 \leq a/A \leq 1{,}2;$$

und

$$3 \leq b/c \leq 9.$$

Alternativ sind ganz besonders bevorzugt Multimetalloxidmassen der allgemeinen Stöchiometrie I solche, bei denen gleichzeitig gilt:

d = 0,04 bis 0,1;
e = 0,8 bis 1,8;

$$0{,}5 \leq A \leq 1;$$

$$0{,}5 \leq a/A \leq 1{,}2;$$

und

$$3 \leq b/c \leq 5.$$

Erfindungsgemäße Multimetalloxidmassen der allgemeinen Stöchiometrie I werden üblicherweise in Substanz (als sogenannte Vollkatalysatoren) zu geometrischen Formkörpern wie z.B. Kugeln, Ringen oder (Voll)Zylindern geformt, oder auch in Gestalt von Schalenkatalysatoren, d.h. mit der Multimetalloxid(aktiv)masse beschichteten vorgeformten inerten Träger(form)körpern, zur Katalyse der jeweiligen heterogen katalysierten Gasphasenpartialoxidation (z.B. von Propen zu Acrolein) eingesetzt. Selbstverständlich können sie aber auch in Pulverform als Katalysatoren für solche Katalyse eingesetzt werden.

Prinzipiell können Multimetalloxid(aktiv)massen der allgemeinen Stöchiometrie I in einfacher Weise dadurch hergestellt werden, dass man von geeigneten Quellen ihrer (insbesondere der von Sauerstoff verschiedenen) elementaren Konstituenten ein möglichst inniges, vorzugsweise feinteiliges, der jeweiligen Stöchiometrie entsprechend zusammengesetztes Trockengemisch erzeugt und dieses, wahlweise nach zuvor erfolgter Formung zu Formkörpern von regelmäßiger oder unregelmäßiger Geometrie, die wahlweise unter Mitverwendung von Formgebungshilfsmitteln erfolgt, bei Temperaturen im Bereich von 350 bis 650 °C kalciniert. Die Kalcination kann sowohl unter Inertgas als auch unter einer oxidativen Atmosphäre wie z.B. Luft (oder ein anderes Gemisch aus Inertgas und molekularem Sauerstoff) sowie auch unter reduzierender Atmosphäre (z.B. ein Gemisch aus Inertgas, $NH_3$, CO und/oder $H_2$) erfolgen oder unter Vakuum. Die Kalcinationsdauer kann einige Minuten bis einige Stunden betragen und nimmt üblicherweise mit der Kalcinationstemperatur ab.

Als Quellen für die elementaren Konstituenten der Multimetalloxidmassen der allgemeinen Stöchiometrie I (der Multimetalloxidaktivmassen I) kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind.

Neben den Oxiden kommen als solche Ausgangsverbindungen (Quellen) vor allem Halogenide, Nitrate, Formiate, Oxalate, Citrate, Acetate, Carbonate, Aminkomplexe, Ammoniumsalze und/oder Hydroxide sowie Hydrate der vorgenannten Salze in Betracht. Verbindungen wie $NH_4OH, (NH_4)CO_3$, $NH_4NO_3$, $NH_aCHO_2$, $CH_3COOH$, $NH_4CH_3CO_2$ und/oder Ammoniumoxalat, die spätestens beim späteren Kalcinieren zu gasförmig entweichenden Verbindungen zerfallen und/oder zersetzt werden können, können in das innige Trockengemisch zusätzlich eingearbeitet werden. Als solche sich beim Kalcinieren zersetzende Substanzen kommen auch organische Materialien, wie z.B. Stearinsäure, Malonsäure, Ammoniumsalze der vorgenannten Säuren, Stärken (z.B. Kartoffelstärke und Maisstärke), Cellulose, gemahlene Nussschalen und feinteiliges Kunststoffmehl (z.B. aus Polyethylen, Polypropylen etc.), in Betracht.

Das innige Vermischen der Ausgangsverbindungen (Quellen) zur Herstellung von Multimetalloxidaktivmassen I kann in trockener oder in nasser Form erfolgen. Erfolgt es in trockener Form, so werden die Ausgangsverbindungen (die Quellen) zweckmäßigerweise als feinteilige Pulver eingesetzt und nach dem Mischen und gegebenenfalls Verdichten zum geometrischen Vorläuferformkörper der Kalcination unterworfen. Vorzugsweise erfolgt das innige Vermischen jedoch in nasser Form.

Erfindungsgemäß vorteilhaft werden dabei die Ausgangsverbindungen in Form von Lösungen und/oder Suspensionen miteinander vermischt und die dabei resultierende nasse Mischung M anschließend zum innigen Trockengemisch getrocknet. Als Lösungs- und/oder Suspendiermittel wird bevorzugt Wasser bzw. eine wässrige Lösung eingesetzt.

Ganz besonders innige Trockengemische werden beim vorstehend beschriebenen Mischverfahren dann erhalten, wenn ausschließlich von in gelöster Form vorliegenden Quellen und/oder von kolloidal gelösten Quellen der elementaren Konstituenten ausgegangen wird. Ganz generell kann eine Ausgangsverbindung Quelle für nur einen oder für mehr als einen elementaren Konstituenten sein. In dementsprechender Weise kann eine vorstehend aufgeführte Lösung bzw. kolloidale Lösung nur einen oder auch mehr als einen elementaren Konstituenten gelöst aufweisen. Bevorzugtes Lösungsmittel ist dabei, wie bereits gesagt, Wasser. Die Trocknung der resultierenden wässrigen Gemische erfolgt vorzugsweise durch Sprühtrocknung.

Wird in vorliegender Schrift von einer Lösung einer Quelle (Ausgangsverbindung, Ausgangssubstanz) in einem Lösungsmittel (z.B. Wasser) gesprochen, so ist dabei der Begriff "Lösen" im Sinn einer molekularen bzw. ionischen Lösung gemeint. Das heißt, die in der Lösung befindliche größte geometrische Einheit der gelösten Ausgangssubstanz (Quelle) weist unabdingbar "molekulare" Ausmaße auf.

Demgegenüber stellen kolloidale Lösungen eine Verbindung zwischen echten (molekularen und/oder ionischen) Lösungen und Suspensionen dar. In diesen kolloiddispersen Systemen befinden sich kleinere Anhäufungen von Molekülen oder Atomen, welche jedoch weder mit dem bloßen Auge, noch mit dem Mikroskop erkennbar sind. Die kolloidale Lösung erscheint optisch völlig klar (wenn auch oft gefärbt), da die in ihr enthaltenen Teilchen nur einen Durchmesser von 1 bis 250 nm (vorzugsweise bis 150 nm und besonders bevorzugt bis 100 nm) haben. Aufgrund der geringen Größe ist eine Abtrennung der kolloidal gelösten Partikel durch konventionelle Filtration nicht möglich. Sie können jedoch durch Ultrafiltration mit Membranen pflanzlichen, tierischen oder künstlichen Ursprungs (z.B. Pergament, Schweinsblase oder Cellophan) von ihrem "Lösungsmittel" abgetrennt werden. Im Gegensatz zu den "optisch leeren" echten (molekularen und/oder ionischen) Lösungen, kann ein Lichtstrahl nicht ohne Ablenkung durch eine kolloidale Lösung hindurch treten. Der Lichtstrahl wird von den kolloidal gelösten Partikeln gestreut und abgelenkt. Um kolloidale Lösungen stabil zu halten und weitergehende Partikelagglomerationen zu verhindern, enthalten sie häufig Netz- und Dispergierhilfsmittel sowie andere Additive zugesetzt.

Beispielsweise kann das Element Silizium (der elementare Konstituent Si) in Form eines Kieselsols zur Herstellung der nassen (z.B. wässrigen) Mischung M eingebracht werden. Kieselsole sind kolloidale Lösungen von amorphem Siliciumdioxid in Wasser. Sie sind wasserflüssig und enthalten keine sedimentierbaren Bestandteile. Ihr $SiO_2$-Gehalt kann bei oft jahrelanger Haltbarkeit (ohne Sedimentation) bis zu 50 Gew.-% und mehr betragen.

Selbstverständlich können in einer zur Herstellung einer nassen (z.B. wässrigen) Mischung M zu verwendenden Lösung wenigstens einer Elementquelle auch molekular und/oder ionisch gelöste Quellen sowie kolloidal gelöste Quellen nebeneinander in Lösung vorliegen.

Eine günstige Mo-Quelle ist im Rahmen der Herstellung erfindungsgemäßer Multimetalloxidaktivmassen I Ammoniumheptamolybdattetrahydrat. Weitere mögliche Mo-Quellen sind Ammoniumorthomolybdat ($(NH_4)_2MoO_4$), Ammoniumdimolybdat ($(NH_4)_2Mo_2O_7$), Ammoniumtetramolybdatdihydrat ($(NH_4)_2Mo_4O_{13}$ x 5 $H_2O$) und Ammoniumdecamolybdatdihydrat ($(NH_4)_4Mo_{10}O_{32}$ x 2 $H_2O$). Grundsätzlich ist aber auch z.B. Molybdantrioxid einsetzbar.

Bevorzugte K-Quelle zur Herstellung erfindungsgemäßer Multimetalloxidmassen I ist KOH (Kaliumhydroxid). Grundsätzlich kann aber auch $KNO_3$ bzw. dessen Hydrat als K-Quelle verwendet werden.

Als Bi-Quelle werden zur Herstellung erfindungsgemäßer Multimetalloxidaktivmassen I vorzugsweise Salze des Bismuts eingesetzt, die das Bi als $Bi^{3+}$ aufweisen. Als solche Salze kommen z.B. Bismut(III)oxid, Bismut(III)oxidnitrat (Bismutsubnitrat), Bismut(III)halogenid (z.B. Fluorid, Chlorid, Bromid, Jodid) und insbesondere Bismut(III)nitratpentahydrat in Betracht.

Erfindungsgemäß bevorzugte Fe-Quelle sind Salze des $Fe^{3+}$, unter denen die verschiedenen Eisen(III)nitrathydrate besonders bevorzugt sind (vgl. z.B. DE-A 102007003076). Besonders bevorzugt wird erfindungsgemäß Eisen(III)nitratnonahydrat als Fe-Quelle eingesetzt. Selbstverständlich können auch Salze des $Fe^{2+}$ als Fe-Quelle im Rahmen einer erfindungsgemäßen Herstellung von Multimetalloxidaktivmassen I verwendet werden.

Erfindungsgemäß vorteilhaft wird zur Herstellung erfindungsgemäßer Multimetalloxidmassen I, bezogen auf die in ihnen enthaltene molare Gesamtmenge an Fe, wenigstens 50 mol-%, besser wenigstens 75 mol-% und bevorzugt wenigstens 95 mol-% in Form einer Fe-Quelle eingebracht, die das Fe als $Fe^{3+}$ aufweist. Auch können Fe-Quellen verwendet werden, die sowohl $Fe^{2+}$ wie auch $Fe^{3+}$ aufweisen.

Erfindungsgemäß geeignete Co-Quellen sind dessen Salze, die das Co als $Co^{2+}$ und/oder $Co^{3+}$ aufweisen. Als solche seien beispielhaft das Kobald(II)nitrathexahydrat, das $Co_3O_4$, das CoO, das Kobalt(II)formiat und das Kobalt(III)nitrat genannt. Die Erstere dieser Quellen ist besonders bevorzugt.

Häufig erfolgt die Herstellung einer nassen (z.B. wässrigen) Mischung M erfindungsgemäß bevorzugt an Luft (vorteilhaft ist die wässrige Mischung M an Luft gesättigt). Dies gilt insbesondere dann, wenn als Kobalt- und Eisen-Quelle Salze des $Co^{2+}$ sowie Salze des $Fe^{2+}$ verwendet werden. Vor allem dann, wenn es sich bei diesen Salzen um die Nitrate und/oder deren Hydrate handelt. Diese Vorteilhaftigkeit fußt nicht zuletzt darauf, dass $Fe^{2+}$ und $Co^{2+}$ im Beisein von $NO_3^-$ durch den molekularen Sauerstoff der Luft wenigstens partiell zu $Fe^{3+}$ bzw. $Co^{3+}$ aufoxidiert werden können.

Wie bereits erwähnt, handelt es sich bei der nassen Mischung M erfindungsgemäß bevorzugt um eine wässrige Mischung M, die mit besonderem Vorteil auf folgende Weise erzeugt wird. Aus wenigstens einer Quelle der Elemente Co, Fe und Bi wird eine wässrige Lösung A hergestellt, deren pH-Wert ≤ 3, vorzugsweise ≤ 2, besonders bevorzugt ≤ 1 und ganz besonders bevorzugt ≤ 0 beträgt. In der Regel beträgt der pH-Wert der wässrigen Lösung A nicht weniger als -2 und liegt besonders vorteilhaft im Bereich -1 bis 0. Bevorzug ist die wässrige Lösung A eine wässrige Lösung der Nitrate bzw. Nitrat-Hydrate von Co, Bi und Fe. Besonders bevorzugt ist die wässrige Lösung A eine wässrige Lösung der Nitrate bzw. Nitrat-Hydrate in wässriger Salpetersäure. Zur Herstellung einer solchen Lösung kommen als Elementquelle auch unmittelbar Lösungen der relevanten Elemente in wässriger Salpetersäure in Betracht.

Aus wenigstens einer Quelle des Elements Mo sowie wahlweise einer oder mehrerer Quellen des Elements K wird eine wässrige Lösung B hergestellt. Der pH-Wert der wässrigen Lösung B beträgt erfindungsgemäß vorteilhaft (auf 25 °C und 1,01 bar bezogen) < 7. Besonders bevorzugt beträgt der pH-Wert der wässrigen Lösung B ≤ 6,5 und ganz besonders vorteilhaft ≤ 6. In der Regel wird der pH-Wert der wässrigen Lösung B ≥ 3 betragen. Günstige erfindungsgemäß zu verwendende Lösungen B weisen einen pH-Wert von 4 bis 6 auf. pH-Werte wässriger Lösungen beziehen sich in dieser Schrift generell (soweit nicht explizit etwas anderes gesagt wird) auf eine Messung bei 25 °C und 1 atm (1,01 bar) mit einer als Einstabmesskette ausgeführten Glaselektrode. Die Eichung derselben erfolgt mittels Pufferlösungen, deren pH-Wert bekannt ist und die in der Nähe des gesuchten Messwertes liegt. Insbesondere eignet sich zur Bestimmung von pH-Werten im erfindungsgemäßen Zusammenhang die Mettler Toledo pH-Elektrode Inpro 4260/425/Pt 100, die eine Einstabmesskette mit integriertem Temperaturfühler Pt 100 zur automatischen Temperaturkompensation ist.

Enthält die wässrige Lösung B K, so wird als dessen Quelle zur Zubereitung der wässrigen Lösung B vorteilhaft KOH verwendet. Bevorzugte Mo-Quelle zur Zubereitung einer wässrigen Lösung B ist Ammoniumheptamolybdattetrahydrat ($(NH_4)_6Mo_7O_{24}$ x 4 $H_2O$).

Der Gesamtgehalt der wässrigen Lösung A an Co, Fe und Bi beträgt erfindungsgemäß zweckmäßig, bezogen auf die Menge des in der wässrigen Lösung A enthaltenen Wassers, 10 bis 25 Gew.-%, vorteilhaft 15 bis 20 Gew.-%.

Der Gesamtgehalt der wässrigen Lösung B an Mo beträgt erfindungsgemäß zweckmäßig, bezogen auf die Menge des in der wässrigen Lösung B enthaltenen Wassers, 3 bis 25 Gew.-%, vorteilhaft 5 bis 15 Gew.-%.

Anschließend werden die wässrige Lösung A und die wässrige Lösung B anwendungstechnisch zweckmäßig miteinander vermischt. Erfindungsgemäß vorteilhaft wird dabei so verfahren, dass die wässrige Lösung A kontinuierlich in die wässrige Lösung B eingerührt wird. Erfindungsgemäß vorteilhaft wird die vorgelegte wässrige Lösung B dabei intensiv gerührt. Der Gesamtgehalt der resultierenden wässrigen Mischung aus wässriger Lösung A und wässriger Lösung B an Mo, Co, Fe und Bi beträgt erfindungsgemäß zweckmäßig, bezogen auf die Menge des in der wässrigen Mischung enthaltenen Wassers, 5 bis 25 Gew.-%, vorteilhaft 8 bis 20 Gew.-%.

Die Temperatur der vorgelegten wässrigen Lösung B sowie der beim Einrühren der wässrigen Lösung A in selbige resultierenden, intensiv gerührten wässrigen Mischung beträgt erfindungsgemäß vorteilhaft (vorzugsweise während des gesamten Mischvorgangs) $\leq 80\ °C$, besser $\leq 70\ °C$, noch besser $\leq 60\ °C$ und bevorzugt $\leq 40\ °C$. In der Regel wird vorgenannte Temperatur $0\ °C$ nicht unterschreiten. Mit Vorteil weist die wässrige, in die Lösung B eingerührte Lösung A die gleiche Temperatur auf wie die vorgelegte Lösung B. Vorzugsweise ist die Temperatur der wässrigen Vorlage über den Verlauf des beschriebenen Einrührprozesses konstant. Zu diesem Zweck kann beispielsweise mit Hilfe eines Wasserbades thermostatisiert werden. Der Arbeitsdruck liegt anwendungstechnisch zweckmäßig bei 1,01 bar (1 atm).

Vorzugsweise wird die wässrige Lösung A innerhalb eines Zeitraums im Bereich von 5 bis 60 Minuten, besonders bevorzugt innerhalb eines Zeitraums von 10 bis 30 Minuten und ganz besonders bevorzugt innerhalb eines Zeitraums von 15 bis 25 Minuten in die vorgelegte wässrige Lösung B eingerührt. Das resultierende wässrige Gemisch wird nachfolgend, vorzugsweise unter Beibehalt der Einrührtemperatur, anwendungstechnisch zweckmäßig 5 bis 60 Minuten, vorteilhaft 10 bis 30 Minuten und besonders vorteilhaft 15 bis 25 Minuten nachgerührt.

Im Wesentlichen hat die Größe des Zeitraums, während dessen die wässrige Lösung A und die wässrige Lösung B zusammengeführt werden, keinen Einfluss auf die Selektivität der im weiteren Verlauf erzeugten Multimetalloxidaktivmasse I. Übermässiges Nachrühren ($\geq 4$ h) mindert die Selektivität. Es hat sich ferner gezeigt, dass die Größe der vorgenannten Zeiträume einen gewissen Einfluss auf die Aktivität der im weiteren Verlauf erzeugten Multimetalloxidaktivmasse I ausüben. So fördert ein langsameres Einrühren der wässrigen Lösung A in die wässrige Lösung B die Aktivität, während ein zu rasches Einrühren der wässrigen Lösung A in die wässrige Lösung B die Aktivität mindert. Letzteres trifft auch auf übermäßiges Nachrühren zu (z.B. $\geq 3$ h, bzw. $\geq 4$ h).

Das Verhältnis V, gebildet aus der in der wässrigen Mischung der wässrigen Lösung A und der wässrigen Lösung B wahlweise enthaltenen molaren Gesamtmenge $n_1$ an $NH_3$ und $NH_4^+$ und der in derselben wässrigen Mischung enthaltenen molaren Gesamtmenge $n_2$ an Mo ($V = n_1 : n_2$) wird erfindungsgemäß vorteilhaft so eingestellt, dass $V \leq 1,5$, vorzugsweise $\leq 1$ und besonders bevorzugt $\leq 6/7$ beträgt. Grundsätzlich kann V auch 0 betragen. Der pH-Wert der wässrigen Mischung aus wässriger Lösung A und wässriger Lösung B beträgt mit Vorteil gleichzeitig $\leq 3$, besser $\leq 2$. In der Regel liegt er bei Werten $\geq 0$.

Enthält die angestrebte Multimetalloxidaktivmasse I den elementaren Konstituenten Si, so wird als Quelle desselben vorteilhaft wässriges Kieselsol (vgl. z.B. DE-A 102006044520) verwendet und dieses in zweckmäßiger Weise in die wässrige Mischung aus wässriger Lösung A und wässriger Lösung B eingerührt, wobei dieser wässrigen Mischung vorab dieses Einrührens in vorteilhafter Weise noch Wasser zugesetzt werden kann. In zweckmäßiger Weise kann sowohl das wässrige Kieselsol als auch das Wasser auf einmal zugesetzt werden. Sowohl die Temperatur des Wassers als auch die Temperatur des wässrigen Kieselsols entspricht dabei in vorteilhafter Weise der Temperatur der wässrigen Mischung aus wässriger Lösung A und wässriger Lösung B. Abschließend wird in zweckmäßiger Weise noch bis zu 30 Minuten nachgerührt. Während des Nachrührens wird die vorgenannte Temperatur vorteilhaft beibehalten. Der $SiO_2$-Gehalt des zugesetzten wässrigen Kieselsols kann 15 bis 60 Gew.-%, oder 20 bis 60 Gew.-%, oder 30 bis 60 Gew.-%, vorzugsweise 40 bis 60 Gew.-% und besonders bevorzugt 45 bis 55 Gew.-% betragen (jeweils bezogen auf sein Gesamtgewicht).

Anstelle die wässrige Lösung B in einem thermostatisierten gerührten Behälter vorzulegen und anschließend unter Rühren in selbigen die wässrige Lösung A zulaufen zu lassen, können auch beide, die wässrige Lösung B und die wässrige Lösung A, dem gerührten Behälter kontinuierlich zugeführt werden (z.B. durch einen "3-Wege-T-Mischer" hindurch). Grundsätzlich kann auch die wässrige Lösung B kontinuierlich in eine vorgelegte wässrige Lösung A eingerührt werden. Diese Verfahrensweise ist jedoch erfindungsgemäß weniger bevorzugt.

In einer alternativen Ausführungsform wird aus Quellen der Elemente Fe und Bi eine wässrige Lösung A* erzeugt. Aus Quellen der Elemente Co und Mo, sowie wahlweise K, wird eine wässrige Lösung B* erzeugt. Nachfolgend werden die wässrige Lösung A* und die wässrige Lösung B* miteinander vermischt (vorzugsweise wird die wässrige Lösung A* in die wässrige Lösung B* eingerührt). Dem resultierenden wässrigen Gemisch von wässriger Lösung A* und wässriger Lösung B* kann dann wieder bedarfsgerecht als Si-Quelle wässriges Kieselsol zugesetzt werden. Hinsichtlich pH-Wert der verschiedenen wässrigen Lösungen, möglicher Quellen der elementaren Konstituenten sowie bezüglich des Verhältnisses V im resultierenden wässrigen Gemisch aus wässriger Lösung A* und wässriger Lösung B* gilt das im Zusammenhang mit den wässrigen Lösungen A, B und deren Gemisch bereits Gesagte in entsprechender Weise.

Während die Herstellung wässriger Lösungen A, B sowie die Mischung derselben, wie bereits erwähnt. vorzugsweise im Beisein von gasförmigem molekularem Sauerstoff (z.B. im Beisein von Luft) erfolgt, hat es sich im Fall der Herstellung der wässrigen Lösungen A*, B* sowie deren Mischung als vorteilhaft erwiesen, unter Ausschluss von molekularem Sauerstoff zu arbeiten.

In der Regel handelt es sich bei der wie beschrieben erhältlichen wässrigen Mischung M um eine wässrige Suspension (vorzugsweise liegen in der wässrigen Mischung M ebenfalls die als vorteilhaft beschriebenen Verhältnisse V vor (enthaltene molare Gesamtmenge an $NH_3 + NH_4^+$ zu enthaltener molarer Menge an Mo); darüber hinaus beträgt auch der pH-Wert der wie beschrieben erhältlichen wässrigen Mischung M vorteilhaft $\leq 3$, in der Regel 0 bis 2). Erfindungsgemäß vorteilhaft enthalten wie beschrieben erhältliche wässrige Mischungen M nicht mehr oder weniger als 60 % der in ihnen enthaltenen Gesamtmenge an Co in im wässrigen Medium gelöster Form (bei derjenigen Temperatur und dem Arbeitsdruck, bei dem die wässrige Mischung M erzeugt wurde). Bevorzugt liegt der vorstehend aufgeführte, im wässrigen Medium der wässrigen Mischung M gelöst vorliegende Anteil AT der in der wässrigen Mischung M insgesamt enthaltenen Menge an Co bei Werten $\leq 50$ % und besonders bevorzugt bei Werten $\leq 40$%, bzw. $\leq 30$ % oder $\leq 20$ %. Der Gesamtgehalt der zu trocknenden (vorzugsweise sprüh zu trocknenden) wässrigen Mischung M an Mo, Co, Fe, Bi und Si beträgt erfindungsgemäß zweckmäßig, bezogen auf die Menge des in der wässrigen Mischung M enthaltenen Wassers, 5 bis 25 Gew.-%, vorteilhaft 8 Bis 20 Gew.-%. In der Regel beträgt AT $\geq 10$ %, oder $\geq 15$ %. Die Überführung der wässrigen Mischung M in ein feinteiliges inniges Trockengemisch erfolgt erfindungsgemäß bevorzugt durch Sprühtrocknung der wässrigen Mischung M. Das heißt, die wässrige Mischung M wird zunächst in feinteilige Tröpfchen zerteilt und selbige daran anschließend getrocknet. Erfindungsgemäß bevorzugt erfolgt die Sprühtrocknung im Heißluftstrom. Grundsätzlich können zur vorgenannten Sprühtrocknung aber auch andere heiße Gase verwendet werden (z.B. Stickstoff oder mit Stickstoff verdünnte Luft sowie sonstige inerte Gase).

Die Sprühtrocknung kann dabei sowohl im Gleichstrom als auch im Gegenstrom der Tröpfchen zum heißen Gas erfolgen. Typische Gaseintrittstemperaturen liegen dabei im Bereich von 250 bis 450 °C, vorzugsweise 270 bis 370 °C. Typische Gasaustrittstemperaturen liegen dabei im Bereich von 100 bis 160 °C. Vorzugsweise erfolgt die Sprühtrocknung im Gegenstrom der Tröpfchen zum heißen Gas.

Selbstverständlich kann die wässrige Mischung M auch durch konventionelles Eindampfen (vorzugsweise bei vermindertem Druck; die Trocknungstemperatur wird im Regelfall 150 °C nicht überschreiten) getrocknet werden. Grundsätzlich kann die Trocknung einer wässrigen Mischung M auch durch Gefriertrocknung erfolgen.

Grundsätzlich kann die getrocknete wässrige Mischung M als solche zu einer erfindungsgemäßen Multimetalloxid(aktiv)masse der allgemeinen Stöchiometrie I kalciniert werden. Insbesondere dann, wenn die Trocknung der wässrigen Mischung M durch Sprühtrocknung erfolgte, ist das resultierende Sprühpulver jedoch für eine unmittelbare Kalcination häufig zu feinteilig. In diesem Fall kann das Sprühpulver z.B. durch nachfolgendes Kompaktieren vergröbert werden. Erfolgt die Kompaktierung trocken, kann vorab der Kompaktierung z.B. feinteiliger Graphit und/oder andere in dieser Schrift genannte Formgebungshilfsmittel (z.B. Gleitmittel und/oder Verstärkungsmittel) unter das Sprühpulver gemischt werden (z.B. mit einem Röhnradmischer). Beispielsweise kann die Kompaktierung mit einem zwei gegenläufige Stahlwalzen aufweisenden Kalander durchgeführt werden. Anschließend kann das Kompaktat zielgerichtet auf die für die ins Auge gefasste Weiterverwendung angemessene Partikelgröße zerkleinert werden. Dies kann in einfachster Weise z.B. dadurch erfolgen, dass das Kompaktat durch ein Sieb mit definierter Maschenweite gedrückt wird.

Die Kompaktierung kann aber grundsätzlich auch feucht erfolgen. Beispielsweise kann das Sprühpulver unter Zusatz von Wasser geknetet werden. Im Anschluss an die Knetung kann die geknetete Masse auf die Folgeverwendung abgestimmt wieder zur gewünschten Feinteiligkeit zerkleinert (vgl. z.B. DE-A 10049873) und getrocknet werden.

Die wie beschrieben erhältlichen feinteiligen Vorläufermaterialien können nun als solche kalciniert und das dabei erhältliche Multimetalloxid(aktiv)massen-I-Pulver als solches zur Katalyse von heterogen katalysierten partiellen Gasphasenoxidationen von z.B. Propen zu Acrolein eingesetzt werden. Alternativ kann das erhaltene Multimetalloxid(aktiv)massen-I-Pulver aber auch zunächst zu Formkörpern von regelmäßiger oder unregelmäßiger Geometrie geformt und die dabei resultierenden Formkörper als Katalysatoren für die heterogen katalysierte partielle Gasphasenoxidation von z.B. Propen zu Acrolein eingesetzt werden (vgl. z.B. DE-A 10063162).

Beispielsweise können aus der Pulverform der Aktivmasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formungshilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse auch durch Aufbringen auf die äußere Oberfläche von vorgeformten inerten Katalysatorträgern erfolgen. Die Beschichtung der Trägerformkörper zur Herstellung solcher Schalenkatalysatoren kann dabei z.B. in einem geeigneten drehbaren Behälter ausgeführt werden, wie es aus der DE-A 10063162, der DE-A 2909671, der EP-A 293859, der EP-A 714700 und der DE-A 4442346 bekannt ist.

Anwendungstechnisch alternativ kann man zur Herstellung von Schalenkatalysatorformkörpern die Beschichtung der Trägerformkörper mit dem nicht kalcinierten Vorläuferpulver selbst vornehmen und die Kalcination erst nach erfolgtem Aufbringen sowie gegebenenfalls Trocknen vornehmen (vgl. z.B. DE-A 10049873).

Die zur Herstellung von Schalenkatalysatoren zu verwendenden Trägerformkörper sind vorzugsweise chemisch inert, d.h., sie greifen in den Ablauf der zu katalysierenden partiellen Gasphasenoxidation des z.B. Propens zu Acrolein im Wesentlichen nicht ein. Als Materialien für die Trägerformkörper kommen erfindungsgemäß insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht (insbesondere Steatit C220 der Fa. CeramTec).

Die Oberfläche des Trägerformkörpers kann sowohl glatt als auch rauh sein. Mit Vorteil ist die Oberfläche des Trägerformkörpers rauh, da eine erhöhte Oberflächenrauhigkeit in der Regel eine erhöhte Haftfestigkeit der aufgebrachten Schale an feinteiliger oxidischer Aktivmasse oder an feinteiliger Vorläufermasse bedingt. Häufig liegt die Oberflächenrauhigkeit $R_z$ des Trägerformkörpers im Bereich von 40 bis 200 $\mu m$, oft im Bereich von 40 bis 100 $\mu m$ (bestimmt gemäß DIN 4768 Blatt 1 mit einem "Hommel Tester für DIN-ISO Oberflächenmaßgrößen" der Fa. Hommelwerke, DE). Ferner kann das Trägermaterial porös oder unporös sein. Zweckmäßigerweise ist das Trägermaterial unporös (das Gesamtvolumen der Poren beträgt auf das Volumen des Trägerformkörpers bezogen vorteilhaft 1 ≤ Vol.-%).

Die Feinheit der auf die Oberfläche des Trägerformkörpers aufzubringenden feinteiligen Masse wird selbstredend an die gewünschte Schalendicke angepasst. Für den Bereich einer Schalendicke von 100 bis 500 $\mu m$ eignen sich z.B. feinteilige Massen, von denen wenigstens 50 % der Pulverpartikel ein Sieb der Maschenweite 1 bis 10 $\mu m$ passieren und deren Anteil an Partikeln mit einer Längstausdehnung (= längste direkte Verbindungslinie zweier auf der Oberfläche befindlicher Punkte) oberhalb von 50 $\mu m$ weniger als 1 % (bezogen auf die Gesamtzahl der Partikel) beträgt. In der Regel entspricht die Verteilung der Längstausdehnungen der Pulverpartikel herstellungsbedingt einer Gaußverteilung.

Zweckmäßigerweise wird zur Beschichtung der Trägerformkörper die Oberfläche derselben und/oder die aufzubringende feinteilige Pulvermasse mit einem flüssigen Bindemittel (z.B. Wasser oder organische Lösungsmittel wie Glyzerin oder deren Gemisch) befeuchtet und der Schalenformkörper nach erfolgtem Aufbringen z.B. mittels heißer Luft wieder getrocknet. Die Schichtdicke der auf dem Trägerformkörper aufgebrachten feinteiligen Pulvermasse wird zweckmäßigerweise im Bereich 10 bis 1000 $\mu m$, bevorzugt im Bereich 100 bis 700 $\mu m$ und besonders bevorzugt im Bereich 300 bis 500 $\mu m$ liegend gewählt. Mögliche Schalendicken sind auch 10 bis 500 $\mu m$ oder 200 bis 300 $\mu m$.

Der Trägerformkörper selbst kann, wie bereits erwähnt, regelmäßig oder unregelmäßig geformt sein, wobei regelmäßig geformte Trägerformkörper wie z.B. Kugeln, Vollzylinder oder Hohlzylinder bevorzugt werden. Erfindungsgemäß geeignet ist z.B. die Verwendung von kugelförmigen Trägerformkörpern, deren Durchmesser 1 bis 8 mm, bevorzugt 4 bis 5 mm beträgt. Geeignet ist aber auch die Verwendung von Zylindern als Trägerformkörper, deren Länge 2 bis 10 mm und deren Außendurchmesser 4 bis 10 mm beträgt. Im Fall von erfindungsgemäß geeigneten Ringen als Trägerformkörper liegt die Wanddicke darüber hinaus üblicherweise bei 1 bis 4 mm. Erfindungsgemäß geeignete Zylinderabmessungen sind auch 3 bis 6 mm (Länge), 4 bis 8 mm (Außendurchmesser) und, im Fall von Ringen, 1 bis 2 mm (Wanddicke). Selbstverständlich kommt als erfindungsgemäß geeignete Ringgeometrie auch in Betracht 2 bis 4 mm (Länge), 4 bis 8 mm (Außendurchmesser) und 1 bis 2 mm (Wanddicke). Erfindungsgemäß markante Trägerringgeometrien sind z.B. 7 mm x 3 mm x 1,5 mm (Außendurchmesser x Länge x Wanddicke) und 5 mm x 3 mm x 1,5 mm (Außendurchmesser x Länge x Wanddicke). Im Einzelnen können Trocknung und/oder thermische Behandlung (Kalcination) nach dem Aufbringen der Schale wie in der DE-A 10063162 sowie der DE-A 10049873 beschrieben durchgeführt werden.

Erfindungsgemäß besonders vorteilhaft wird jedoch so vorgegangen, dass aus feinteiliger Vorläufermasse (aus dem feinteiligen innigen Trockengemisch der Quellen der elementaren Konstituenten) anwendungstechnisch zweckmäßig durch Verdichten (Komprimieren oder Kompaktieren) Formkörper von regelmäßiger oder unregelmäßiger Geometrie geformt und daran anschließend durch thermische Behandlung (Kalcination) in sogenannte Vollkatalysatorformkörper überführt werden.

Diese Vorgehensweise ist insbesondere dann bevorzugt, wenn das innige Vermischen der Ausgangsverbindungen (Quellen) der relevanten elementaren Konstituenten der Multimetalloxidmasse I zum feinteiligen innigen Trockengemisch in trockener Form erfolgt (vgl. z.B. WO 2008/087116 und DE-A 102008042060).

Als weitere feinteilige Formgebungshilfsmittel können dabei beispielsweise wieder Gleitmittel wie z.B. Graphit, Ruß, Polyethylenglykol, Polyacrylsäure, Stearinsäure, Stärke, Mineralöl, Pflanzenöl, Wasser, Bortrifluorid und/oder Bornitrid zugegeben werden. Ferner kommen als Formgebungshilfsmittel Verstärkungsmittel wie Mikrofasern aus Glas, Asbest, Siliciumcarbid oder Kaliumtitanat in Betracht, die sich nach Beendigung der Formgebung durch Verdichten förderlich auf den Zusammenhalt des erhaltenen Komprimats (des resultierenden Formkörpers) auswirken. Eine Mitverwendung von Gleitmitteln im Rahmen einer entsprechenden Formgebung findet sich z.B. in den Schriften DE-A 102007004961, WO 2008/087116, WO 2005/030393, US-A 2005/0131253, WO 2007/017431, DE-A 102007005606 und in der DE-A 102008040093 beschrieben.

Erfindungsgemäß bevorzugt wird ausschließlich feinteiliger Graphit als Gleitmittel mit verwendet. Dabei kommen als zu verwendende feinteilige Graphite insbesondere jene in Betracht, die in den Schriften WO 2005/030393, US-A 2005/0131253, WO 2008/087116 und DE-A 102007005606 empfohlen werden. Dies gilt insbesondere für jene Graphite, die in diesen Schriften in den Beispielen und Vergleichsbeispielen eingesetzt werden. Ganz besonders bevorzugte Graphite sind Asbury 3160 und Asbury 4012 der Firma Asbury Graphite Mills, Inc., New Jersey 08802, USA und Timrex®T44 der Firma Timcal Ltd., 6743 Bodio, Schweiz.

Bezogen auf das Gewicht der zu formenden feinteiligen Vorläufermasse kann diese z.B. bis zu 15 Gew.-% an feinteiligem Gleitmittel (z.B. Graphit) enthalten. Meist liegt der Gleitmittelgehalt in der zu formenden feinteiligen Vorläufermasse (in dem feinteiligen innigen Trockengemisch) jedoch bei ≤ 9 Gew.-%, vielfach bei ≤ 5 Gew.-%, oft bei ≤ 4 Gew.-%; dies insbesondere dann, wenn das feinteilige Gleitmittel Graphit ist. In der Regel beträgt die vorgenannte Zusatzmenge ≥ 0,5 Gew.-%, meist ≥ 2,5 Gew.-%.

Im Regelfall erfolgt die Verdichtung der feinteiligen, wahlweise Formgebungshilfsmittel umfassenden, Vorläufermasse (des feinteiligen innigen Trockengemischs) zur gewünschten Geometrie des Formkörpers (des geometrischen Katalysatorvorläuferformkörpers) durch Einwirkung äußerer Kräfte (Druck) auf die Vorläufermasse. Der dabei anzuwendende Formgebungsapparat bzw. die dabei anzuwendende Formgebungsmethode unterliegt keiner Beschränkung.

Beispielsweise kann die verdichtende Formgebung durch Strangpressen, Tablettieren oder Extrudieren erfolgen. Dabei wird die feinteilige Vorläufermasse (das feinteilige innige Trockengemisch) vorzugsweise anfasstrocken eingesetzt. Sie kann jedoch z.B. bis zu 10 % ihres Gesamtgewichts Substanzen zugesetzt enthalten, die bei Normalbedingungen (25 °C, 1 atm (1,01 bar)) flüssig sind. Auch kann die feinteilige Vorläufermasse (das feinteilige innige Trockengemisch) feste Solvate (z.B. Hydrate) enthalten, die solche flüssigen Substanzen in chemisch und/oder physikalisch gebundener Form aufweisen. Selbstverständlich kann die feinteilige Vorläufermasse auch völlig frei von solchen Substanzen sein.

Erfindungsgemäß bevorzugtes Formgebungsverfahren durch Verdichten der feinteiligen Vorläufermasse (des feinteiligen innigen Trockengemischs) ist die Tablettierung. Die Grundzüge des Tablettierens sind z.B. in "Die Tablette", Handbuch der Entwicklung, Herstellung und Qualitätssicherung, W.A.Ritschel und A.Bauer-Brandl, 2. Auflage, Edition Verlag Aulendorf, 2002 beschrieben und in völlig entsprechender Weise auf ein erforderungsgemäßes Tablettierungsverfahren übertragbar.

Mit Vorteil wird eine erfindungsgemäße Tablettierung wie in den Schriften WO 2005/030393, DE-A 102008040093, DE-A 102008040094 und WO 2007/017431 beschrieben durchgeführt. Die die Tablettiermaschine umgebende Temperatur beträgt dabei normalerweise 25 °C. Anwendungstechnisch zweckmäßig liegen die Partikeldurchmesser der zu verdichtenden Vorläufermasse (des feinteiligen innigen Trockengemischs), wahlweise als Ergebnis einer Vorvergröberung durch Kompaktieren, im Bereich 100 bis 2000 $\mu$m, bevorzugt 150 bis 1500 $\mu$m, besonders bevorzugt 400 bis 1250 $\mu$m, oder 400 bis 1000 $\mu$m, oder 400 bis 800 $\mu$m (vorab der Verdichtung eingemischtes Formgebungshilfsmittel ist dabei nicht mitberücksichtigt).

Ebenso wie der zur Verdichtung zu verwendende Formgebungsapparat bzw. die dabei anzuwendende Formgebungsmethode, unterliegt auch die angestrebte Geometrie der resultierenden Formkörper beim erfindungsgemäßen Verfahren keiner Beschränkung. D.h., die beim Verdichten erzeugten Katalysatorvorläuferformkörper können sowohl regelmäßig als auch unregelmäßig geformt sein, wobei regelmäßig geformte Formkörper in der Regel erfindungsgemäß bevorzugt werden.

Beispielsweise kann der Katalysatorvorläuferformkörper Kugelgeometrie aufweisen. Dabei kann der Kugeldurchmesser z.B. 2 bis 10 mm, oder 4 bis 8 mm betragen. Die Geometrie des Katalysatorvorläuferformkörpers kann aber auch vollzylindrisch oder hohlzylindrisch (ringförmig) sein.

In beiden Fällen können Außendurchmesser und Höhe (Länge) z.B. 2 bis 10 mm, oder 2 bis 8 mm, oder 3 bis 8 mm betragen.

Im Fall von Hohlzylindern (Ringen) ist in der Regel eine Wandstärke (Wanddicke) von 1 bis 3 mm zweckmäßig. Selbstverständlich kommen als Katalysatorvorläufergeometrien aber auch alle diejenigen Geometrien in Betracht, die in der WO 02/062737 offenbart und empfohlen werden. Im Fall von Vollzylindern kann der Außendurchmesser auch 1 bis 10 mm betragen.

Die im Rahmen einer wie beschrieben durchzuführenden Verdichtung von feinteiliger Vorläufermasse (feinteiligem innigen Trockengemisch) angewandten Formgebungsdrucke werden erfindungsgemäß vorteilhaft 50 kg/cm$^2$ bis 5000 kg/cm$^2$ betragen. Vorzugsweise betragen die Formgebungsdrucke 200 bis 3500 kg/cm$^2$, besonders bevorzugt 600 bis 2500 kg/cm$^2$.

Insbesondere im Fall von ringförmigen Vorläuferformkörpern (die in der Literatur unabhängig von ihrer Form auch als Grünlinge bezeichnet werden) soll die formgebende erfindungsgemäße Verdichtung erfindungsgemäß vorteilhaft so durchgeführt werden, dass die Seitendruckfestigkeit SD des resultierenden Formkörpers (vgl. DE-A 102008040093, DE-A 102008040094 und WO 2005/030393) die Relation 12 N ≤ SD ≤ 35 N, vorzugsweise 15 N ≤ SD ≤ 30 N, und besonders bevorzugt 19 N ≤ SD ≤ 30 N erfüllt.

Die experimentelle Bestimmung der Seitendruckfestigkeit wird dabei wie in den Schriften WO 2005/030393 sowie WO 2007/017431 beschrieben durchgeführt. Selbstverständlich sind ringähnliche Grünlinge, wie sie die DE-A 102008040093 empfiehlt, erfindungsgemäß ganz besonders bevorzugt. Die Stirnfläche von ringförmigen oder ringähnlichen Formkörpern kann beim beschriebenen Herstellverfahren erfindungsgemäßer Grünlinge sowohl gekrümmt als auch nicht gekrümmt sein (vgl. insbesondere DE-A 102007004961, EP-A 184790 und die DE-A 102008040093). Bei der Ermittlung der Höhe solcher geometrischer Formkörper wird eine solche Krümmung nicht berücksichtigt.

Erfindungsgemäß besonders vorteilhafte Ringgeometrien von durch Verdichten von feinteiliger Vorläufermasse (feinteiligem innigem Trockengemisch) erhältlichen Formkörpern erfüllen die Bedingung Höhe/Außendurchmesser = H/A = 0,3 bis 0,7. Besonders bevorzugt ist H/A = 0,4 bis 0,6. Weiterhin ist es für erfindungsgemäße ringförmige bzw. ringähnliche Grünlinge günstig, wenn das Verhältnis I/A (wobei I der Innendurchmesser der Ringgeometrie ist) 0,3 bis 0,7, vorzugsweise 0,4 bis 0,7 beträgt.

Besonders vorteilhaft sind vorgenannte Ringgeometrien, wenn sie gleichzeitig eines der vorteilhaften H/A-Verhältnisse und eines der vorteilhaften I/A-Verhältnisse aufweisen. Solche möglichen Kombinationen sind z.B. H/A = 0,3 bis 0,7 und I/A = 0,3 bis 0,8 oder 0,4 bis 0,7. Alternativ kann H/A 0,4 bis 0,6 und I/A gleichzeitig 0,3 bis 0,8 oder 0,4 bis 0,7 betragen. Ferner ist es für die relevanten Ringgeometrien günstig, wenn H 2 bis 6 mm und vorzugsweise 2 bis 4 mm beträgt. Weiterhin ist es vorteilhaft, wenn A bei den Ringen 4 bis 8 mm, vorzugsweise 4 bis 6 mm beträgt. Die Wandstärke von erfindungsgemäß bevorzugten Grünling-Ringgeometrien beträgt 1 bis 1,5 mm.

Mögliche erfindungsgemäße Ringgeometrien sind somit (A x H x I) 5 mm x 2 mm x 2 mm, oder 5 mm x 3 mm x 2 mm, oder 5 mm x 3 mm x 3mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm, oder 7 mm x 7 mm x 3 mm, 7 mm x 3 mm x 4 mm, oder 7 mm x 7 mm x 4 mm.

Generell erweist es sich für das erfindungsgemäße Verfahren als vorteilhaft, wenn jede der zur Herstellung der aus den Quellen der von Sauerstoff verschiedenen elementaren Konstituenten der Multimetalloxidmasse I zu erzeugenden feinteiligen innigen Trockengemische mit verwendeten Quellen im Verlauf der Herstellung des feinteiligen innigen Trockengemischs einen Zerteilungsgrad durchläuft, der dadurch gekennzeichnet ist, dass sein Durchmesser $d^Q_{90} \leq 5\ \mu m$ beträgt.

Das Erfordernis $d^Q_{90} \leq 5\ \mu m$ ist grundsätzlich dann erfüllt, wenn man eine Quelle in einem Lösungsmittel auflöst (der Begriff "Lösen" ist dabei im Sinn einer molekularen bzw. ionischen Lösung gemeint). Dies ist dadurch bedingt, dass beim Lösen einer Quelle (Ausgangsverbindung) in einem Lösungsmittel die Quelle im Lösungsmittel molekular bzw. ionisch zerteilt wird. Das heißt, die in der Lösung befindliche größte geometrische Einheit der gelösten Ausgangssubstanz (Quelle) weist unabdingbar "molekulare" Ausmaße auf, die damit in notwendiger Weise wesentlich kleiner als 5 $\mu m$ sind (wie bereits gesagt, kann eine Ausgangsverbindung Quelle für mehr als ein Element sein, und eine Lösung kann mehr als eine Quelle gelöst aufweisen).

Das Erfordernis $d^Q_{90} \leq 5\ \mu m$ ist aber auch dann erfüllt, wenn sich eine Quelle eines Elements in einem Lösungsmittel in kolloidaler Lösung befindet, da die in ihr gelöst enthaltenen Einheiten nur einen Durchmesser von 1 bis 250 nm haben, weshalb der zugehörige $d^Q_{90}$ in notwendiger Weise $\leq 5\ \mu m$ beträgt.

Das Erfordernis $d^Q_{90} \leq 5\ \mu m$ ist aber auch dann erfüllt, wenn eine Quelle z.B. trocken auf diese Partikelgröße zerkleinert wird (z.B. durch Mahlen).

Der Partikeldurchmesser $d^Q_{90}$ bezieht sich dabei auf die Partikeldurchmesserverteilung des Trockenpulvers, die wie folgt zu ermitteln ist.

Das feinteilige Pulver wird über eine Dispergierrinne in den Trockendispergierer Scirocco 2000 (der Firma Malvern Instruments Ltd., Worcestershire WR14 1AT, United Kingdom) geführt, dort mit Druckluft trocken dispergiert und im Freistrahl in die Messzelle geblasen. In dieser wird dann nach ISO 13320 mit dem Laserbeugungsspektrometer Malvern Mastersizer S (ebenfalls von der Firma Malvern Instruments Ltd.) die volumenbezogene Partikeldurchmesserverteilung bestimmt.

Ein auf eine solche Partikeldurchmesserverteilung bezogener Partikeldurchmesser $d_x$ ist dabei so definiert, dass X % des Gesamtpartikelvolumens aus Partikeln mit diesem oder einem kleineren Durchmesser bestehen. Das heißt, (100 - X) % des Gesamtpartikelvolumens bestehen aus Partikeln mit einem Durchmesser $> d_x$. Wird in dieser Schrift nicht ausdrücklich etwas anderes erwähnt, beziehen sich Partikeldurchmesserbestimmungen und daraus entnommene $d_x$ wie z.B. $d_{90}$, $d_{50}$ und $d_{10}$ auf einen bei der Bestimmung angewandten (die Stärke der Dispergierung des Trockenpulvers während der Messung bestimmenden) Dispergierdruck von 2 bar absolut. $d^Q_{90}$ ist ein solcher Partikeldurchmesser $d_{90}$ einer pulverförmigen Quelle.

Alle in dieser Schrift bezüglich eines Röntgendiffraktogramms gemachten Angaben beziehen sich auf ein unter Anwendung von Cu-$K_\alpha$-Strahlung als Röntgenstrahlung erzeugtes Röntgendiffraktogramm (Diffraktometer Theta-Theta Bruker D8 Advance, Röhrenspannung: 40 kV, Röhrenstrom: 40 mA, Aperturblende V20 (variabel), Streustrahlblende V20 (variabel), Detektorblende (0,1 mm), Maßintervall (2Θ = 2 Theta): 0,02°, Messzeit je Schritt: 2,4 s, Detektor: Si-Halbleiterdetektor).

Alle Angaben in dieser Schrift zu spezifischen Oberflächen von Feststoffen beziehen sich auf Bestimmungen nach DIN 66131 (Bestimmung der spezifischen Oberfläche von Feststoffen durch Gasadsorption ($N_2$) nach Brunauer-Emmert-Teller (BET)), soweit nicht ausdrücklich etwas anderes erwähnt wird.

**EP 2 731 715 B1**

Alle Angaben in dieser Schrift zu Porengesamtvolumina sowie zu Porendurchmesserverteilungen auf diese Porengesamtvolumina beziehen sich auf Bestimmungen mit der Methode der Quecksilberporosimetrie in Anwendung des Gerätes Auto Pore 9500 der Fa. Micromeritics GmbH, D-41238 Mönchengladbach (Bandbreite 0,003 - 300 $\mu$m).

Erfindungsgemäß vorteilhaft weisen Vollkatalysatorvorläuferformkörper eine möglichst geringe Restfeuchtigkeit auf. Dies insbesondere dann, wenn das innige Mischen der verschiedenen Quellen der von Sauerstoff verschiedenen elementaren Konstituenten der Multimetalloxidmasse I nass erfolgte (insbesondere dann, wenn es unter Ausbildung einer wässrigen Mischung M erfolgte).

Erfindungsgemäß bevorzugt liegt der Restfeuchtegehalt erfindungsgemäß vorteilhafter Grünlinge bei Werten ≤ 10 Gew.-%, besser ≤ 8 Gew.-%, noch besser ≤ 6 Gew.-%, und am Besten ≤ 4 Gew.-% oder ≤ 2 Gew.-% (die Restfeuchtebestimmung kann dabei wie in "Die Bibliothek der Technik", Band 229, "Thermogravimetrische Materialfeuchtebestimmung", Grundlagen und praktische Anwendungen, Horst Nagel, verlag moderne industrie, beschrieben erfolgen (z.B. mit Hilfe eines Computrac MAX 5000 XL von Arizona Instruments)).

Gehen die Grünlinge auf eine wässrige Mischung M zurück (so dass ihr Restfeuchtegehalt aus Wasser besteht), erfolgt die Restfeuchtebestimmung andwendungstechnisch zweckmäßig mit Mikrowellen (z.B. mit dem Mikrowellensystem LB 456 von BERTHOLD TECHNOLOGIES).

Die Mikrowelle durchstrahlt bei dieser Verfahrensweise mit sehr geringer Leistung (0,1 mW) das zu untersuchende Material (letzteres erfährt infolge der vergleichsweise geringen Leistung im Wesentlichen keine Änderung seiner Temperatur). Die Materialbestandteile werden dadurch unterschiedlich stark polarisiert. Als Reaktion verliert die Mikrowelle an Geschwindigkeit und Energie. Dabei ist der Einfluss von Wassermolekülen wesentlich größer als der Einfluss anderer Bestandteile, was die selektive Bestimmung von Restwassergehalten ermöglicht. Dies rührt daher, dass Wassermoleküle auf Grund ihrer Größe und ihrer Dipoleigenschaft einem elektromagnetischen Wechselfeld im Mikrowellenfrequenzbereich durch Dipolausrichtung besonders gut zu folgen vermögen. Dabei absorbieren sie Energie und verändern mit ihren elektrischen Eigenschaften das elektromagnetische Wechselfeld. Auf dieser Feldschwächung und Feldveränderung beruht das Messprinzip. Beispielsweise kann über der Sensorfläche eines Planarsensors ein schwaches Mikrowellenfeld aufgebaut und durch Abscannen der Mikrowellenfrequenz permanent die Resonanzfrequenz des Sensorsystems analysiert werden. Bringt man nun ein wasserhaltiges Messgut über den Sensor, so verschiebt sich die Resonanzfrequenz und ihre Amplitude wird gedämpft. Beides, Dämpfung und Resonanzfrequenz-Verschiebung steigen mit zunehmender Wassermenge, also auch mit zunehmender Schüttdichte des Messguts. Allerdings ist dabei das Verhältnis aus Frequenzverschiebung und Dämpfung ein dichteunabhängiges Maß für den prozentualen Wasseranteil und damit der Schlüssel für die Feuchtemessung. Das Verhältnis bildet den so genannten Mikrowellen-Feuchtemesswert, der die Gesamtfeuchte repräsentiert. Da es sich bei dem Mikrowellen-Resonanzverfahren um ein indirektes Feuchtemessverfahren handelt, ist eine Kalibrierung notwendig. Bei einer solchen Kalibriermessung werden mit dem Sensor Materialproben mit definierter Feuchte gemessen. Die Verknüpfung der Mikrowellen-Feuchtemesswerte mit den zugehörigen definierten absoluten Materialfeuchten bildet dann die Kalibrierung des Messsystems. Die Messgenauigkeit beträgt üblicherweise ± 0,1 % Feuchte (beispielsweise kann die Wasserfeuchte mit einem Online-Feuchte-Messgerät PMD300PA von Sartorius ermittelt werden).

Vor diesem Hintergrund sollte bereits eine Sprühtrocknung einer nassen (z.B. wässrigen) Mischung M so durchgeführt werden, dass das resultierende Sprühpulver einen möglichst geringen Restfeuchtegehalt aufweist.

Erfindungsgemäß hergestellte Grünlinge sollten unter Berücksichtigung des eben adressierten Gesichtspunktes möglichst unter Ausschluss von (Luftfeuchtigkeit aufweisender) Umgebungsluft gelagert werden (vorzugsweise erfolgt die Lagerung bis zur Kalcinierung unter wasserfreiem Inertgas bzw. unter vorab getrockneter Luft).

Erfindungsgemäß vorteilhaft wird bereits die Formung von feinteiligem innigem Trockengemisch unter Ausschluss von (Luftfeuchtigkeit aufweisender) Umgebungsluft (z.B. unter $N_2$-Atmosphäre) durchgeführt.

Die Kalcination der Grünlinge (bzw. generell von feinteiligem Vorläuferpulver oder von mit diesem beschichteten Trägerformkörpern) erfolgt normalerweise bei Temperaturen, die wenigstens 350 °C erreichen oder in der Regel überschreiten. Normalerweise wird im Rahmen der Kalcination die Temperatur von 650 °C jedoch nicht überschritten (der Begriff der Kalcinationstemperatur meint dabei in dieser Schrift die im Kalcinationsgut vorliegende Temperatur). Erfindungsgemäß vorteilhaft wird im Rahmen der Kalcination die Temperatur 600 °C, bevorzugt die Temperatur von 550 °C und häufig die Temperatur von 500 °C nicht überschritten. Ferner wird im Rahmen der vorstehenden Kalcination vorzugsweise die Temperatur von 380 °C, mit Vorteil die Temperatur von 400 °C, mit besonderem Vorteil die Temperatur von 420 °C und ganz besonders bevorzugt die Temperatur von 440 °C überschritten. Dabei kann die Kalcination in ihrem zeitlichen Ablauf auch in mehrere Abschnitte gegliedert sein.

Erfahrungsgemäß vorteilhaft erfolgt vorab der Kalcination eine thermische Behandlung bei Temperaturen von ≥ 120 °C und < 350 °C, vorzugsweise ≥ 150 °C und ≤ 320 °C, besonders bevorzugt ≥ 220 °C und ≤ 290 °C.

Eine solche thermische Behandlung wird anwendungstechnisch zweckmäßig so lange durchgeführt, bis die innerhalb der thermisch zu behandelnden Masse enthaltenen, sich unter den Bedingungen der thermischen Behandlung zu gasförmigen Verbindungen zersetzenden, Bestandteile weitgehend (vorzugsweise vollständig) zu gasförmigen Verbindungen zersetzt worden sind (der diesbezüglich erforderliche Zeitaufwand kann z.B. 3 h bis 10 h, häufig 4 h bis 8 h betragen). Dies ist in der Regel dann der Fall, wenn einerseits die in der sich daran anschließend zu kalcinierenden Masse enthaltene molare Menge an von Metallionen verschiedenen Kationen, bezogen auf die enthaltene molare Gesamtmenge an Kationen, $\leq$ 20 mol-% (vorzugsweise $\leq$ 10 mol-%) und andererseits die in derselben Masse enthaltene molare Menge an von $O^{2-}$ verschiedenen Anionen, bezogen auf die enthaltene molare Gesamtmenge an Anionen, ebenfalls $\leq$ 20 mol-% (vorzugsweise $\leq$ 10 mol-%) beträgt.

Erfindungsgemäß günstige Temperaturfenster für die Endkalcinationstemperatur liegen daher im Temperaturbereich 400 °C bis 600 °C, bzw. vorzugsweise im Temperaturbereich 420 bis 550 °C, oder besonders bevorzugt im Temperaturbereich 400 bis 500 °C.

Die Gesamtdauer der Kalcination erstreckt sich in der Regel auf mehr als 10 h. Meist werden im Rahmen der Kalcination Behandlungsdauern von 45 h, bzw. von 25 h nicht überschritten. Oft liegt die Gesamtkalcinationsdauer unterhalb von 20 h. Grundsätzlich ist bei höheren Kalcinationstemperaturen in der Regel eine kürzere Kalcinationsdauer ausreichend als bei niedrigeren Kalcinationstemperaturen.

In einer erfindungsgemäß vorteilhaften Ausführungsform der Kalcination werden 500 °C nicht überschritten und die Kalcinationsdauer im Temperaturfenster $\geq$ 430 °C und $\leq$ 500 °C erstreckt sich auf > 10 bis $\leq$ 20 h.

Die gesamte thermische Behandlung (einschließlich einer Zersetzungsphase) einer Vorläufermasse (z.B. eines Grünlings) kann sowohl unter Intertgas als auch unter einer oxidativen Atmosphäre wie z.B. Luft (oder ein sonstiges Gemisch aus Inertgas und molekularem Sauerstoff) sowie unter reduzierender Atmosphäre (z.B. ein Gemisch aus Inertgas, $NH_3$, CO und/oder $H_2$ oder unter Methan, Acrolein, Methacrolein) erfolgen. Selbstredend kann die thermische Behandlung auch unter Vakuum ausgeführt werden. Auch kann die Atmosphäre über den Verlauf der thermischen Behandlung variabel gestaltet werden.

Erfindungsgemäß bevorzugt erfolgt die thermische Behandlung (insbesondere die Kalcinationsphase) in einer oxidierende Atmosphäre. Anwendungstechnisch zweckmäßig besteht diese überwiegend aus stehender oder (bevorzugt) bewegter Luft (besonders bevorzugt wird die thermisch zu behandelnde Masse (das Kalcinationsgut) von einem Luftstrom durchströmt). Die oxidierende Atmosphäre kann jedoch ebenso aus einem stehenden oder bewegten Gemisch aus z.B. 25 Vol.-% $N_2$ und 75 Vol.-% Luft, oder 50 Vol.-% $N_2$ und 50 Vol.-% Luft, oder 75 Vol.-% $N_2$ und 25 Vol.-% Luft bestehen (eine Behandlungsatmosphäre aus 100 Vol.-% $N_2$ ist ebenfalls möglich).

Prinzipiell kann die thermische Behandlung (z.B. die Kalcination) der Vorläufermasse (z.B. der Grünlinge) in den unterschiedlichsten Ofentypen wie z.B. beheizbare Umluftkammern (Umluftöfen, z.B. Umluftschachtöfen), Hordenöfen, Drehrohröfen, Bandkalcinierern oder Schachtöfen durchgeführt werden. Erfindungsgemäß vorteilhaft erfolgt die thermische Behandlung (z.B. die Kalcination) in einer Bandkalciniervorrichtung, wie sie die DE-A 10046957 und die WO 02/24620 empfehlen. Eine Heißpunktausbildung innerhalb des zu behandelnden Gutes (innerhalb des Kalcinationsgutes) wird dabei weitestgehend dadurch vermieden, dass mit Hilfe von Ventilatoren durch ein das Kalcinationsgut tragendes gasdurchlässiges Förderband erhöhte Volumenströme an Kalcinationsatmosphäre durch das Kalcinationsgut gefördert werden.

Im Rahmen der wie beschrieben durchzuführenden thermischen Behandlung der Vorläufermassen (z.B. der Grünlinge), können mit verwendete Formgebungshilfsmittel sowohl im resultierenden Katalysatorformkörper erhalten bleiben, als auch durch thermische und/oder chemische Zersetzung zu gasförmigen Verbindungen (z.B. CO, $CO_2$) wenigstens teilweise gasförmig aus diesen entweichen. Im Katalysatorformkörper verbleibende Formgebungshilfsmittel wirken im Rahmen einer katalytischen Verwendung desselben in selbigem im Wesentlichen ausschließlich als die Multielementoxid-I-Aktivmasse verdünnend. Grundsätzlich kann die thermische Behandlung diesbezüglich wie in der US 2005/0131253 beschrieben erfolgen.

In typischer Weise betragen die Seitendruckfestigkeiten von erfindungsgemäß wie beschrieben erhältlichen ringförmigen Vollkatalysatorformkörpern 5 bis 15 N, häufig 6 bis 13 N oder 8 bis 11 N.

Die spezifische (BET) Oberfläche von erfindungsgemäßen Multimetalloxid(aktiv)massen I (insbesondere, wenn sie, wie vorstehend beschrieben, zu ringförmigen Vollkatalysatoren ausgeformt sind) beträgt anwendungstechnisch vorteilhaft 2 bis 20 bzw. bis 15 $m^2/g$, vorzugsweise 3 bis 10 $m^2/g$ und besonders bevorzugt 4 bis 8 $m^2/g$. Das dazugehörige Porengesamtvolumen liegt dabei erfindungsgemäß vorteilhaft im Bereich von 0,1 bis 1 $cm^3/g$ bzw. bis 0,8 $cm^3/g$, vorzugsweise im Bereich 0,1 bis 0,5 $cm^3/g$ und besonders bevorzugt im Bereich 0,2 bis 0,4 $cm^3/g$.

Trägt man auf der Abszisse den Porendurchmesser in $\mu$m auf und auf der Ordinate den Logarithmus des differentiellen Beitrags in $cm^3/g$ des jeweiligen Porendurchmesser zum Porengesamtvolumen in $cm^3/g$, so zeigen erfindungsgemäß besonders günstige Multimetalloxid(aktiv)massen I (insbesondere, wenn sie, wie vorstehend beschrieben, zu ringförmigen Vollkatalysatoren ausgeformt sind) in der Regel eine monomodale Verteilung (weist lediglich ein Maximum auf). Beträgt dabei der Beitrag von Poren mit einem Porenradius $\leq$ 0,1 $\mu$m zum Porengesamtvolumen $\leq$ 0,05 $cm^3/g$, resultieren besonders gute Gesamtzielproduktselektivitäten (z.B. im Fall einer heterogen katalysierten partiellen Oxidation von Propen zu Acrolein und/oder Acrylsäure). Für den Fall, dass der Beitrag solcher vergleichsweise enger Poren zum Gesamtporenvolumen > 0,05 $cm^3/g$ beträgt, kann durch eine Erhöhung der Kalcinationsdauer und/oder der Kalcinationstemperatur eine erfindungsgemäß vorteilhafte Minderung dieses Beitrags bewirkt werden.

Darüber hinaus erweist es sich für eine erhöhte Gesamtzielproduktselektivität als vorteilhaft, wenn der Beitrag von Poren mit einem Porenradius im Bereich von 0,2 bis 0,4 μm zum Porengesamtvolumen, bezogen auf das Porengesamtvolumen, ≥ 70 Vol.-%, vorteilhaft ≥ 75 Vol.-%, besonders vorteilhaft ≥ 85 Vol.-%, vorzugsweise ≥ 90 Vol.-%, besonders bevorzugt ≥ 95 Vol.-% beträgt.

Selbstverständlich kann die erfindungsgemäße Multimetalloxid(aktiv)masse der allgemeinen Stöchiometrie I auch mit inerten Materialien verdünnt zur Katalyse von heterogen katalysierten partiellen Gasphasenoxidationen eingesetzt werden. Als solche inerten Verdünnungsmaterialien eignen sich unter anderen bei hohen Temperaturen gebrannte und dadurch vergleichsweise an Poren arme Elementoxide wie Aluminiumoxid, Siliciumoxid, Thoriumdioxid und Zirkondioxid. Aber auch feinteiliges Siliciumcarbid oder feinteilige Silikate wie Magnesium- und Aluminiumsilikat oder Steatit können zum vorgenannten Zweck eingesetzt werden. Anwendungstechnisch vorteilhaft wird man dabei z.B. so vorgehen, dass die kalcinierte Multimetalloxid(aktiv)masse der allgemeinen Stöchiometrie I zu einem feinteiligen Pulver gemahlen wird. Dieses wird dann anwendungstechnisch zweckmäßig mit feinteiligem Verdünnungsmaterial vermischt und das dabei resultierende Mischpulver unter Anwendung eines in dieser Schrift vorgestellten Formgebungsverfahrens (vorzugsweise durch Tablettieren) zu einem geometrischen Formkörper geformt. Durch nachfolgendes nochmaliges Kalcinieren wird selbiger dann in den zugehörigen Katalysatorformkörper transformiert. Selbstredend kann das feinteilige inerte Verdünnungsmaterial aber z.B. auch bereits in eine nasse (z.B. wässrige) Mischung M vorab deren Trocknung eingearbeitet werden. Des Weiteren kann eine Einarbeitung von feinteiligem inertem Verdünnungsmaterial in ein feinteiliges Trockengemisch von Quellen der elementaren Konstituenten der Multimetalloxidmasse I hinein erfolgen. Solche Vorgehensweisen sind jedoch erfindungsgemäß weniger bevorzugt.

Insbesondere gemäß den beschriebenen vorteilhaften Herstellverfahren erzeugte Multimetalloxid(aktiv)massen der allgemeinen Stöchiometrie I (bzw. selbige umfassende Vollkatalysatorformkörper) zeichnen sich dadurch aus, dass sie im Wesentlichen keine lokalen Zentren aus Elementoxiden (z.B. Eisenoxid oder Kobaltoxid) aufweisen. Vielmehr sind diese Elemente weitestgehend Bestandteil komplexer, gemischter, Fe, Co und Mo enthaltender Oxomolybdate. Dies hat sich im Hinblick auf eine erfindungsgemäß angestrebte Minimierung von erfindungsgemäß unerwünschter Vollverbrennung organischer Reaktionsgasgemischbestandteile im Rahmen der relevanten heterogen katalysierten Partialoxidationen als günstig erwiesen.

Erfindungsgemäße Multimetalloxid(aktiv)massen der allgemeinen Stöchiometrie I eignen sich als Aktivmassen zur Katalyse von heterogen katalysierten partiellen Gasphasenoxidationen von 3 bis 6 C-Atome aufweisenden Alkanen, Alkanolen, Alkenen und/oder Alkenalen (unter Partialoxidationen sollen dabei in dieser Schrift insbesondere solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation). Der Begriff der partiellen Oxidation soll in dieser Schrift aber auch die oxidative Dehydrierung und die partielle Ammoxidation, d.h., eine partielle Oxidation im Beisein von Ammoniak, umfassen.

Besonders eignen sich erfindungsgemäße Multimetalloxid(aktiv)massen der allgemeinen Stöchiometrie I zur Katalyse der heterogen katalysierten partiellen Gasphasenoxidation von Propen zu Acrolein, von iso-Buten zu Methacrolein sowie zur Katalyse der heterogen katalysierten partiellen Gasphasenammoxidation von Propen zu Acrylnitril sowie von iso-Buten zu Methacrylnitril.

Wie bereits erwähnt bildet die heterogen katalysierte partielle Gasphasenoxidation von Propen (iso-Buten und/oder tert. Butanol) zu Acrolein (Methacrolein) die erste Stufe einer zweistufigen heterogen katalysierten partiellen Gasphasenoxidation von Propen (iso-Buten und/oder tert. Butanol) zu Acrylsäure (Methacrylsäure), wie es beispielhaft in der WO 2006/42459 beschrieben ist.

Eine mit einer heterogen katalysierten partiellen Gasphasenoxidation von Propen (iso-Buten) zu Acrolein (Methacrolein) einhergehende Nebenproduktbildung an Acrylsäure (Methacrylsäure) ist daher in der Regel nicht unerwünscht und subsumiert normalerweise unter der erwünschten Wertproduktbildung.

Das Vorgenannte gilt insbesondere für erfindungsgemäße ringförmige, Multimetalloxidmassen der allgemeinen Stöchiometrie I umfassende, Vollkatalysatorformkörper.

Die heterogen katalysierte Partialoxidation (insbesondere diejenige von Propen zu Acrolein) kann dabei z.B. wie in den Schriften DE-A 102007004961, WO 02/49757, WO 02/24620, DE-A 102008040093, WO 2005/030393, EP-A 575897, WO 2007/082827, WO 2005/113127, WO 2005/047224, WO 2005/042459, WO 2007/017431, DE-A 102008042060, WO 2008/087116, DE-A 102010048405, DE-A 102009047291, DE-A 102008042064, DE-A 102008042061 und DE-A 102008040094 beschrieben durchgeführt werden.

Dabei erweisen sich die in dieser Schrift individualisiert hervorgehobenen Ringgeometrien der wie beschrieben erhältlichen ringförmigen Vollkatalysatoren insbesondere auch dann als vorteilhaft, wenn die Belastung der Katalysatorbeschickung mit im Reaktionsgaseingangsgemisch enthaltenem Propen, iso-Buten und/oder tert. Butanol (bzw. dessen Methylether) ≥ 130 Nl/l Katalysatorbeschickung • h beträgt (Vor- und/oder Nachschüttungen aus reinem Inertmaterial werden bei Belastungsbetrachtungen in dieser Schrift nicht als zur Katalysatorbeschickung gehörig betrachtet; das Volumen der Katalysatorbeschickung ist im Übrigen deren Schüttvolumen im Reaktor befindlich).

Die Vorteilhaftigkeit von wie beschrieben erhältlichen ringförmigen Vollkatalysatorformkörpern (bzw. sonstigen Multimetalloxid(aktiv)massen der allgemeinen Stöchiometrie I aufweisenden Katalysatoren (Katalysatorformkörpern) liegt aber auch dann vor, wenn die vorgenannte Belastung der Katalysatorbeschickung $\geq$ 140 Nl/l·h, oder $\geq$ 150 Nl/l·h, oder $\geq$ 160 Nl/l·h beträgt. Im Normalfall wird die vorgenannte Belastung der Katalysatorbeschickung $\leq$ 600 Nl/l·h, häufig $\leq$ 500 Nl/l·h, vielfach $\leq$ 400 Nl/l·h oder $\leq$ 350 Nl/l·h betragen. Belastungen im Bereich von $\geq$ 160 Nl/l·h bis $\leq$ 300 bzw. $\leq$ 250 oder $\leq$ 200 Nl/l·h sind besonders zweckmäßig.

Unter der Belastung eines Katalysatorfestbetts mit Reaktionsgaseingangsgemisch wird in dieser Schrift die Menge an Reaktionsgaseingangsgemisch in Normlitern (= Nl; das Volumen in Litern, das die entsprechende Reaktionsgaseingangsgemischmenge bei Normalbedingungen, d.h., bei 0 °C und 1 atm (1,01 bar) einnehmen würde) verstanden, die dem Katalysatorfestbett, bezogen auf das Volumen seiner Schüttung (Schüttungsabschnitte aus reinem Inertmaterial werden nicht mit einbezogen), d.h., auf sein Schüttvolumen pro Stunde, zugeführt wird (-> Einheit = Nl/l·h).

Die Belastung kann auch nur auf einen Bestandteil des Reaktionsgaseingangsgemischs bezogen sein (z.B. nur auf die partiell zu oxidierende organische Ausgangsverbindung). Dann ist es die Volumenmenge dieses Bestandteils (z.B. der organischen Ausgangsverbindung der Partialoxidation), die dem Katalysatorfestbett, bezogen auf das Volumen seiner Schüttung, pro Stunde zugeführt wird.

Selbstverständlich können erfindungsgemäß erhältliche Katalysatoren (z.B. ringförmige Vollkatalysatorformkörper) als Katalysatoren für die Partialoxidation von Propen zu Acrolein bzw. von iso-Buten und/oder tert. Butanol (bzw. dessen Methylether) zu Methacrolein auch bei Belastungen der Katalysatorbeschickung mit der partiell zu oxidierenden Ausgangsverbindung von $\leq$ 130 Nl/l·h, oder $\leq$ 120 Nl/l·h, oder $\leq$ 110 Nl/l·h, in erfindungsgemäß vorteilhafter Weise betrieben werden. In der Regel wird diese Belastung jedoch bei Werten $\geq$ 60 Nl/l·h, oder $\geq$ 70 Nl/l·h, oder $\geq$ 80 Nl/l·h liegen.

Prinzipiell kann die Belastung der Katalysatorbeschickung mit der partiell zu oxidierenden Ausgangsverbindung (Propen, iso-Buten und/oder tert. Butanol (bzw. dessen Methylether)) über zwei Stellschrauben eingestellt werden:

a) die Belastung der Katalysatorbeschickung mit Reaktionsgaseingangsgemisch (das Reaktionsgasgemisch, das dem Katalysatorfestbett zugeführt wird),

und/oder

b) den Gehalt des Reaktionsgaseingangsgemischs mit der partiell zu oxidierenden Ausgangsverbindung.

Die erfindungsgemäß erhältlichen Katalysatoren (z. B. ringförmigen Vollkatalysatorformkörper) eignen sich insbesondere auch dann, wenn bei oberhalb von 130 Nl/l·h liegenden Belastungen der Katalysatorbeschickung mit der partiell zu oxidierenden organischen Verbindung die Belastungseinstellung vor allem über die vorgenannte Stellschraube a) erfolgt.

Der Propenanteil (iso-Butenanteil bzw. tert. Butanolanteil (bzw. der Methyletheranteil)) im Reaktionsgaseingangsgemisch wird im Regelfall (d.h., im Wesentlichen unabhängig von der Belastung) 4 bis 20 Vol.-%, häufig 5 bis 15 Vol.-%, oder 5 bis 12 Vol.-%, oder 5 bis 8 Vol.-% betragen (jeweils bezogen auf das (den) Gesamtvolumen(strom) des Reaktionsgaseingangsgemischs).

Häufig wird man das Gasphasenpartialoxidationsverfahren der mit den wie beschrieben erhältlichen Katalysatoren (z. B. ringförmige Vollkatalysatorformkörper oder sonstige geometrische Katalysatorformkörper) katalysierten Partialoxidation (im Wesentlichen unabhängig von der Belastung) bei einem partiell zu oxidierende (organische) Verbindung (z.B. Propen): Sauerstoff : indifferente Gase (einschließlich Wasserdampf) Volumenverhältnis im Reaktionsgaseingangsgemisch von 1:(1,0 bis 3,0):(5 bis 25), vorzugsweise 1:(1,5 bis 2,3):(10 bis 20), durchführen.

Unter indifferenten Gasen (oder auch Inertgasen) werden dabei solche Gase verstanden, die im Verlauf der Partialoxidation zu wenigstens 95 mol-%, vorzugsweise zu wenigstens 98 mol-% chemisch unverändert erhalten bleiben.

Bei den vorstehend beschriebenen Reaktionsgaseingangsgemischen kann das indifferente Gas zu $\geq$ 20 Vol.-%, oder zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zu $\geq$ 80 Vol.-%, oder zu $\geq$ 90 Vol.-%, oder zu $\geq$ 95 Vol.-% aus molekularem Stickstoff bestehen.

Bei Belastungen der Katalysatorbeschickung mit der partiell zu oxidierenden organischen Verbindung von $\geq$ 150 Nl/l·h ist jedoch die Mitverwendung von inerten Verdünnungsgasen wie Propan, Ethan, Methan, Pentan, Butan, $CO_2$, CO, Wasserdampf und/oder Edelgasen für das Rektionsgaseingangsgemisch empfehlenswert (jedoch nicht zwingend). Generell können diese inerten Gase und ihre Gemische aber auch bereits bei geringeren Belastungen der Katalysatorbeschickung mit der partiell zu oxidierenden organischen Verbindung eingesetzt werden. Auch kann Kreisgas als Verdünnungsgas mitverwendet werden. Unter Kreisgas wird das Restgas verstanden, das verbleibt, wenn man aus dem Produktgasgemisch der Partialoxidation die Zielverbindung im Wesentlichen selektiv abtrennt. Dabei ist zu berücksichtigen, dass die Partialoxidationen zu Acrolein oder Methacrolein mit den erfindungsgemäß erhältlichen z. B. ringförmigen Katalysatorformkörpern nur die erste Stufe einer zweistufigen Partialoxidation zu Acrylsäure oder Methacrylsäure als den eigentlichen Zielverbindungen sein können, so dass die Kreisgasbildung dann meist erst nach der zweiten Stufe erfolgt. Im Rahmen einer solchen zweistufigen Partialoxidation wird in der Regel das Produktgasgemisch der ersten Stufe als solches, gegebenenfalls nach Abkühlung und/oder Sekundärsauerstoffzugabe (in der Regel als Luft), der zweiten Partialoxidationsstufe zugeführt.

Bei der Partialoxidation von Propen zu Acrolein, unter Anwendung der wie beschrieben erhältlichen Katalysatoren (z. B. ringförmigen Katalysatorformkörpern), kann eine typische Zusammensetzung des Reaktionsgaseingangsgemischs gemessen am Reaktoreingang (unabhängig von der gewählten Belastung) z.B. die nachfolgenden Komponenten enthalten:

| | |
|---|---|
| 6 bis 6,5 Vol.-% | Propen, |
| 1 bis 3,5 Vol.-% | $H_2O$, |
| 0,2 bis 0,5 Vol.-% | CO, |
| 0,6 bis 1,2 Vol.-% | $CO_2$, |
| 0,015 bis 0,04 Vol.-% | Acrolein, |
| 10,4 bis 11,3 Vol.-% | $O_2$, und |
| als Restmenge ad 100 Vol.-% molekularer Stickstoff; | |

oder:

| | |
|---|---|
| 5,6 Vol.-% | Propen, |
| 10,2 Vol.-% | Sauerstoff, |
| 1,2 Vol.-% | $CO_x$, |
| 81,3 Vol.-% | $N_2$, und |
| 1,4 Vol.-% | $H_2O$. |

Erstere Zusammensetzungen eignen sich insbesondere bei Propenbelastungen von $\geq$ 130 Nl/l·h und letztere Zusammensetzung insbesondere bei Propenbelastungen < 130 Nl/l·h, insbesondere $\leq$ 100 Nl/l·h des Katalysatorfestbetts.

Als alternative Zusammensetzungen des Reaktionsgaseingangsgemischs kommen für eine Propenpartialoxidation zu Acrolein (unabhängig von der gewählten Belastung) solche in Betracht, die nachfolgende Komponentengehalte aufweisen:

| | |
|---|---|
| 4 bis 25 Vol.-% | Propen, |
| 6 bis 70 Vol.-% | Propan, |
| 5 bis 60 Vol.-% | $H_2O$, |
| 8 bis 65 Vol.-% | $O_2$, und |
| 0,3 bis 20 Vol.-% | $H_2$; |

oder

| | |
|---|---|
| 4 bis 25 Vol.-% | Propen, |
| 6 bis 70 Vol.-% | Propan, |
| 0 bis 60 Vol.-% | $H_2O$, |
| 8 bis 16 Vol.-% | $O_2$, |
| 0 bis 20 Vol.-% | $H_2$, |
| 0 bis 0,5 Vol.-% | CO, |
| 0 bis 1,2 Vol.-% | $CO_2$, |
| 0 bis 0,04 Vol.-% | Acrolein, |
| und als Restmenge ad 100 Vol.-% im Wesentlichen $N_2$; | |

oder

| | |
|---|---|
| 50 bis 80 Vol.-% | Propan, |
| 0,1 bis 20 Vol.-% | Propen, |
| 0 bis 10 Vol.-% | $H_2$, |
| 0 bis 20 Vol.-% | $N_2$, |

(fortgesetzt)

| | |
|---|---|
| 5 bis 15 Vol.-% | H$_2$O, und |

soviel molekularer Sauerstoff, dass das molare Verhältnis von Sauerstoffgehalt zu Propengehalt 1,5 bis 2,5 beträgt.

oder

| | |
|---|---|
| 6 bis 9 Vol.-% | Propen, |
| 8 bis 18 Vol.-% | molekularer Sauerstoff, |
| 6 bis 30 Vol.-% | Propan, und |
| 32 bis 72 Vol.-% | molekularer Stickstoff. |

Das Reaktionsgaseingangsgemisch einer heterogen katalysierten partiellen Propenoxidation mit erfindungsgemäßen Katalysatoren zu Acrolein kann aber auch wie folgt zusammengesetzt sein:

| | |
|---|---|
| 4 bis 15 Vol.-% | Propen, |
| 1,5 bis 30 Vol.-% | (häufig 6 bis 15 Vol.-%) Wasser, |
| ≥ 0 bis 10 Vol.-% | (vorzugsweise ≥ 0 bis 5 Vol.-%) von Propen, Wasser, Sauerstoff und Stickstoff verschiedene Bestandteile, soviel molekularer Sauerstoff, dass das molare Verhältnis von enthaltenem molekularem Sauerstoff zu enthaltenem molekularem Propen 1,5 bis 2,5 beträgt, und als Restmenge bis zur 100 Vol.-% Gesamtmenge molekularer Stickstoff. |

Eine andere mögliche Reaktionsgaseingangsgemischzusammensetzung kann enthalten:

| | |
|---|---|
| 6,0 Vol.-% | Propen, |
| 60 Vol.-% | Luft, und |
| 34 Vol.-% | H$_2$O. |

Alternativ können auch Reaktionsgaseingangsgemische der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 für eine erfindungsgemäße Propenpartialoxidation zu Acrolein verwendet werden.

Auch eignen sich die wie beschrieben erhältlichen erfindungsgemäßen Katalysatoren, z. B. ringförmige Katalysatorformkörper, für die Verfahren der DE-A 10246119 bzw. DE-A 10245585.

Weitere erfindungsgemäß geeignete Reaktionsgaseingangsgemische können im nachfolgenden Zusammensetzungsraster liegen:

| | |
|---|---|
| 7 bis 11 Vol.-% | Propen, |
| 6 bis 12 Vol.-% | Wasser, |
| ≥ 0 bis 5 Vol.-% | von Propen, Wasser, Sauerstoff und Stickstoff verschiedener Bestandteile, |

soviel molekularer Sauerstoff, dass das molare Verhältnis von enthaltenem molekularem Sauerstoff zu enthaltenem Propen 1,6 bis 2,2 beträgt, und

als Restmenge bis zur 100 Vol.-% Gesamtmenge molekularer Stickstoff.

Im Fall von Methacrolein als Zielverbindung kann das Reaktionsgaseingangsgemisch insbesondere auch wie in der DE-A 44 07 020 beschrieben zusammengesetzt sein.

Die Reaktionstemperatur für eine erfindungsgemäße heterogen katalysierte Propenpartialoxidation zu Acrolein liegt bei Verwendung der wie beschrieben erhältlichen erfindungsgemäßen Katalysaoren (z. B. ringförmige Katalysatorformkörper) häufig bei 300 bis 450 °C, oder bis 400 °C, bzw. bis 380 °C. Das Gleiche trifft im Fall von Methacrolein als Zielverbindung zu.

Der Reaktionsdruck liegt für die vorgenannten Partialoxidationen in der Regel bei 0,5 bis bzw. 1,5 bis 3 bzw.

bis 4 bar (gemeint sind in dieser Schrift, soweit nicht ausdrücklich etwas anderes erwähnt ist, stets Absolutdrucke).

Die Gesamtbelastung der Katalysatorbeschickung mit Reaktionsgaseingangsgemisch beläuft sich bei den vorgenannten Partialoxidationen typisch auf 1000 bis 10000 Nl/l·h, meist auf 1500 bis 5000 Nl/l·h und oft auf 2000 bis 4000 Nl/l·h.

Als im Reaktionsgaseingangsgemisch zu verwendendes Propen kommen vor allem polymer grade Propen und chemical grade Propen in Betracht, wie es z. B. die

DE-A 102 32 748 beschreibt.

Als Sauerstoffquelle wird normalerweise Luft eingesetzt.

Die Partialoxidation in Anwendung der wie beschrieben erhältlichen erfindungsgemäßen Katalysatoren (z. B. den ringförmigen Katalysatorformkörpern) kann im einfachsten Fall z. B. in einem Einzonen-Vielkontaktrohr-Festbettreaktor durchgeführt werden, wie ihn die DE-A 44 31 957, die EP-A 700 714 und die EP-A 700 893 beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Eine typische Kontaktrohrlänge beläuft sich z. B. auf 3,20 m. Anwendungstechnisch zweckmäßig beläuft sich die im Rohrbündelbehälter untergebrachte Anzahl an Kontaktrohren auf wenigstens 1000, vorzugsweise auf wenigstens 5000. Häufig beträgt die Anzahl der im Reaktionsbehälter untergebrachten Kontaktrohre 15000 bis 35000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Die Partialoxidation kann aber auch in einem Mehrzonen (z. B. "Zwei-Zonen")-Vielkontaktrohr-Festbettreaktor durchgeführt werden, wie es die DE-A 199 10 506, die DE-A 103 13 213, die DE-A 103 13 208 und die EP-A 1 106 598 insbesondere bei erhöhten Belastungen der Katalysatorbeschickung mit der partiell zu oxidierenden organischen Verbindung empfehlen. Eine typische Kontaktrohrlänge im Fall eines Zweizonen-Vielkontaktrohr-Festbettreaktors beträgt 3,50 m. Alles andere gilt im Wesentlichen wie beim Einzonen-Vielkontaktrohr-Festbettreaktor beschrieben. Um die Kontaktrohre, innerhalb derer sich die Katalysatorbeschickung befindet, wird in jeder Temperierzone ein Wärmeaustauschmittel geführt. Als solche eignen sich z. B. Schmelzen von Salzen wie Kaliumnitrat, Kaliumnitrit, Natriumnitrit und/oder Natriumnitrat, oder von niedriger schmelzenden Metallen wie Natrium, Quecksilber sowie Legierungen verschiedener Metalle. Die Fließgeschwindigkeit des Wärmeaustauschmittels innerhalb der jeweiligen Temperierzone wird in der Regel so gewählt, dass die Temperatur des Wärmeaustauschmittels von der Eintrittsstelle in die Temperaturzone bis zur Austrittsstelle aus der Temperaturzone um 0 bis 15 °C, häufig 1 bis 10 °C, oder 2 bis 8 °C, oder 3 bis 6 °C ansteigt.

Die Eingangstemperatur des Wärmeaustauschmittels, das, über die jeweilige Temperierzone betrachtet, im Gleichstrom oder im Gegenstrom zum Reaktionsgasgemisch geführt werden kann, wird vorzugsweise wie in den Schriften EP-A 1 106 598,

DE-A 199 48 523, DE-A 199 48 248, DE-A 103 13 209, EP-A 700 714,

DE-A 103 13 208, DE-A 103 13 213, WO 00/53557, WO 00/53558, WO 01/36364, WO 00/53557 sowie den anderen in dieser Schrift als Stand der Technik zitierten Schriften empfohlen gewählt. Innerhalb der Temperierzone wird das Wärmeaustauschmittel bevorzugt mäanderförmig geführt. In der Regel verfügt der Vielkontaktrohr-Festbettreaktor zusätzlich über Thermorohre zur Bestimmung der Gastemperatur im Katalysatorbett. In zweckmäßiger Weise wird der Innendurchmesser der Thermorohre und der Durchmesser der innen liegenden Aufnahmehülse für das Thermoelement so gewählt, dass das Verhältnis von Reaktionswärme entwickelndem Volumen zu Wärme abführender Oberfläche bei Thermorohren und Arbeitsrohren gleich oder nur geringfügig unterschiedlich ist.

Der Druckverlust sollte bei Arbeitsrohren und Thermorohren, bezogen auf gleiche GHSV, gleich sein. Ein Druckverlustausgleich beim Thermorohr kann z.B. durch Zusatz von versplittetem Katalysator zu den Katalysatorformkörpern erfolgen. Dieser Ausgleich erfolgt zweckmäßig über die gesamte Thermorohrlänge homogen. Im Übrigen kann die Befüllung von Thermorohren wie in der EP-A 873783 beschrieben gestaltet werden.

Zur Bereitung der Katalysatorbeschickung in den Kontaktrohren können, wie bereits erwähnt, nur wie beschrieben erhältliche erfindungsgemäße Katalysatoren (z. B. die ringförmigen Katalysatorformkörper) oder z. B. auch weitgehend homogene Gemische aus wie beschrieben erhältlichen z. B. ringförmigen Katalysatorformkörpern und keine Aktivmasse aufweisenden, sich bezüglich der heterogen katalysierten partiellen Gasphasenoxidation im Wesentlichen inert verhaltenden Formkörpern verwendet werden. Als Materialien für solche inerten Formkörper kommen z. B. poröse oder unporöse Aluminiumoxide, Siliciumdioxid, Zirkondioxid, Siliciumcarbid, Silikate wie Magnesium- oder Aluminiumsilikat und/oder Steatit (z.B. vom Typ C220 der Fa. CeramTec, DE) in Betracht.

Die Geometrie solcher inerter Verdünnungsformkörper kann im Prinzip beliebig sein. D.h., es können beispielsweise Kugeln, Polygone, Vollzylinder oder auch, wie z. B. im Fall ringförmiger Katalysatorformkörper, Ringe sein. Häufig wird man als inerte Verdünnungsformkörper solche wählen, deren Geometrie derjenigen der mit ihnen zu verdünnenden Katalysatorformkörper entspricht. Längs der Katalysatorbeschickung können aber auch die Geometrie des Katalysatorformkörpers gewechselt oder Katalysatorformkörper unterschiedlicher Geometrie in weitgehend homogener Abmischung eingesetzt werden. In einer weniger bevorzugten Vorgehensweise kann auch die Aktivmasse des Katalysatorformkörpers längs der Katalysatorbeschickung verändert werden.

Ganz generell wird, wie bereits erwähnt, die Katalysatorbeschickung mit Vorteil so gestaltet, dass die volumenspezifische (d. h., die auf die Einheit des Volumens normierte) Aktivität in Strömungsrichtung des Reaktionsgasgemischs entweder konstant bleibt oder zunimmt (kontinuierlich, sprunghaft oder stufenförmig).

Eine Verringerung der volumenspezifischen Aktivität kann in einfacher Weise z. B. dadurch erzielt werden, dass man eine Grundmenge von erfindungsgemäß einheitlich hergestellten z. B. ringförmigen Katalysatorformkörpern mit inerten Verdünnungsformkörpern homogen verdünnt. Je höher der Anteil der Verdünnungsformkörper gewählt wird, desto geringer ist die in einem bestimmten Volumen der Beschickung enthaltene Aktivmasse bzw. Katalysatoraktivität. Eine Verringerung kann aber auch dadurch erzielt werden, dass man die Geometrie der erfindungsgemäß erhältlichen Katalysatorformkörper so verändert, dass die in der Einheit des Reaktionsrohrinnenvolumens enthaltene Aktivmassenmenge kleiner wird.

Für die heterogen katalysierten Gasphasenpartialoxidationen mit wie beschrieben erhältlichen erfindungsgemäßen ringförmigen Vollkatalysatorformkörpern wird die Katalysatorbeschickung vorzugsweise entweder auf der gesamten Länge einheitlich mit nur einem Typ von Vollkatalysatorringformkörpern gestaltet oder wie folgt strukturiert. Am Reaktoreingang wird auf einer Länge von 10 bis 60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge der Katalysatorbeschickung, ein im Wesentlichen homogenes Gemisch aus erfindungsgemäß erhältlichem ringförmigem Vollkatalysatorformkörper und inertem Verdünnungsformkörper (wobei beide vorzugsweise im Wesentlichen die gleiche Geometrie aufweisen) platziert, wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d. h., z. B. auf einer Länge von 1,00 bis 3,00 m oder von 1,00 bis 2,70 m, bevorzugt 1,40 bis 3,00 m, oder 2,00 bis 3,00 m) entweder eine nur in geringerem Umfang (als im ersten Abschnitt) verdünnte Schüttung des wie beschrieben erhältlichen erfindungsgemäßen ringförmigen Vollkatalysatorformkörpers, oder, ganz besonders bevorzugt, eine alleinige (unverdünnte) Schüttung desselben erfindungsgemäßen ringförmigen Vollkatalysatorformkörpers, der auch im ersten Abschnitt verwendet worden ist. Natürlich kann über die gesamte Beschickung auch eine konstante Verdünnung gewählt werden. Auch kann im ersten Abschnitt nur mit einem erfindungsgemäß erhältlichen ringförmigen Vollkatalysatorformkörper von geringer, auf seinen Raumbedarf bezogener, Aktivmassendichte und im zweiten Abschnitt mit einem erfindungsgemäß erhältlichen ringförmigen Vollkatalysatorformkörper mit hoher, auf seinen Raumbedarf bezogener, Aktivmassendichte beschickt werden (z.B. 6,5 mm x 3 mm x 4,5 mm [A x H x I] im ersten Abschnitt, und 5 x 2 x 2 mm im zweiten Abschnitt).

Insgesamt werden bei einer mit den wie beschrieben erhältlichen erfindungsgemäßen (z. B. ringförmigen) Katalysatorformkörpern als Katalysatoren durchgeführten Partialoxidation zur Herstellung von Acrolein oder Methacrolein die Katalysatorbeschickung, das Reaktionsgasausgangsgemisch, die Belastung und die Reaktionstemperatur in der Regel so gewählt, dass beim einmaligen Durchgang des Reaktionsgasgemischs durch die Katalysatorbeschickung ein Umsatz der partiell zu oxidierenden organischen Verbindung (Propen, iso-Buten, tert.-Butanol bzw. dessen Methylether) von wenigstens 90 mol-%, oder wenigstens 92 mol-%, vorzugsweise von wenigstens 94 mol-% resultiert. Die Selektivität der Acrolein- bzw. Methacroleinbildung wird dabei regelmäßig $\geq$ 80 mol-%, bzw. $\geq$ 85 mol-% betragen. In natürlicher Weise werden dabei möglichst geringe Heißpunkttemperaturen angestrebt.

Abschließend sei festgehalten, dass wie beschrieben erhältliche erfindungsgemäße ringförmige Vollkatalysatorformkörper auch ein vorteilhaftes Bruchverhalten bei der Reaktorbefüllung aufweisen.

Die Inbetriebnahme einer(s) frischen, erfindungsgemäß erhältliche geometrische Katalysatorformkörper enthaltenden, Katalysatorbeschickung (Katalysatorfestbetts) kann z. B. wie in der DE-A 103 37 788 oder wie in der DE-A 102009047291 beschrieben erfolgen.

Die Formierung erfindungsgemäß erhältlicher geometrischer Katalysatorformkörper kann dadurch beschleunigt werden, dass man sie bei im Wesentlichen gleichbleibendem Umsatz unter erhöhter Belastung der Katalysatorbeschickung mit Reaktionsgaseingangsgemisch durchführt.

Im Übrigen sind erfindungsgemäß erhältliche Multimetalloxidmassen der allgemeinen Stöchiometrie I und selbige als Aktivmasse aufweisende Katalysatoren ganz generell zur Katalyse der Gasphasenpartialoxidation eines 3 bis 6 (d.h., 3, 4, 5 oder 6) C-Atome enthaltenden Alkanols, Alkanals, Alkens, Alkans und Alkenals zu z.B. olefinisch ungesättigten Aldehyden und/oder Carbonsäuren sowie den entsprechenden Nitrilen, und für gasphasenkatalytisch oxidative Dehydrierungen der vorgenannten 3, 4, 5 oder 6 C-Atome enthaltenden organischen Verbindungen geeignet.

Die großtechnische Herstellung von erfindungsgemäßen ringförmigen Vollkatalysatorformkörpern erfolgt anwendungstechnisch zweckmäßig wie in den Deutschen Offenlegungsschriften DE-A 102008040093 und DE-A 102008040094 beschrieben.

Damit umfasst vorliegende Anmeldung insbesondere die folgenden erfindungsgemäßen Ausführungsformen:

1. Mo, Bi und Fe enthaltende Multimetalloxidmassen der allgemeinen Stöchiometrie I,

$$Mo_{12}Bi_aCo_bFe_cK_dSi_eO_x \qquad (I),$$

in der die Variablen folgende Bedeutung haben:

a = 0,5 bis 1,
b = 7 bis 8,5,
c = 1,5 bis 3,0,
d = 0 bis 0,15,
e = 0 bis 2,5 und
x = eine Zahl, die durch die Wertigkeit und Häufigkeit der von Sauerstoff verschiedenen Elemente in I bestimmt wird,
und die folgenden Bedingungen erfüllen:

Bedingung 1: $12 - b - 1,5 \cdot c = A$,
und
$0,5 \leq A \leq 1,5$;

Bedingung 2: $0,2 \leq a/A \leq 1,3$; und

Bedingung 3: $2,5 \leq b/c \leq 9$.

2. Multimetalloxidmasse gemäß Ausführungsform 1, deren stöchiometrischer Koeffizient d 0,04 bis 0,1 beträgt.

3. Multimetalloxidmasse gemäß Ausführungsform 1 oder 2, deren stöchiometrischer Koeffizient d 0,05 bis 0,08 beträgt.

4. Multimetalloxidmasse gemäß einer der Ausführungsformen 1 bis 3, deren stöchiometrischer Koeffizient e 0,5 bis 2 beträgt.

5. Multimetalloxidmasse gemäß einer der Ausführungsformen 1 bis 4, deren stöchiometrischer Koeffizient e 0,8 bis 1,8 beträgt.

6. Multimetalloxidmasse gemäß einer der Ausführungsformen 1 bis 5, deren stöchiometrischer Koeffizient e 1 bis 1,6 beträgt.

7. Multimetalloxidmasse gemäß einer der Ausführungsformen 1 bis 6, die die Bedingung 1, $0,5 \leq A \leq 1,25$ , erfüllt.

8. Multimetalloxidmasse gemäß einer der Ausführungsformen 1 bis 7, die die Bedingung 1, $0,5 \leq A \leq 1$, erfüllt.

9. Multimetalloxidmasse gemäß einer der Ausführungsformen 1 bis 8, die die Bedingung 2, $0,3 \leq a/A \leq 1,2$ , erfüllt.

10. Multimetalloxidmasse gemäß einer der Ausführungsformen 1 bis 9, die die Bedingung 2, $0,4 \leq a/A \leq 1,2$, erfüllt.

11. Multimetalloxidmasse gemäß einer der Ausführungsformen 1 bis 10, die die Bedingung 2, $0,5 \leq a/A \leq 1,2$ , erfüllt.

12. Multimetalloxidmasse gemäß einer der Ausführungsformen 1 bis 11, die die Bedingung 3, $3 \leq b/c \leq 9$ , erfüllt.

13. Multimetalloxidmasse gemäß einer der Ausführungsformen 1 bis 12, die die Bedingung 3, $3 \leq b/c \leq 7$, erfüllt.

14. Multimetalloxidmasse gemäß einer der Ausführungsformen 1 bis 13, die die Bedingung 3, $3 \leq b \leq 5$, erfüllt.

15. Multimetalloxidmasse gemäß einer der Ausführungsformen 1 bis 14, deren spezifische Oberfläche 2 bis 20 $m^2$/g beträgt.

16. Multimetalloxidmasse gemäß einer der Ausführungsformen 1 bis 15, deren spezifische Oberfläche 2 bis 15 $m^2$/g beträgt.

17. Multimetalloxidmasse gemäß einer der Ausführungsformen 1 bis 16, deren spezifische Oberfläche 3 bis 10 m$^2$/g beträgt.

18. Multimetalloxidmasse gemäß einer der Ausführungsformen 1 bis 17, deren spezifische Oberfläche 4 bis 8 m$^2$/g beträgt.

19. Multimetalloxidmasse gemäß einer der Ausführungsformen 1 bis 18, deren Porengesamtvolumen 0,1 bis 1 cm$^3$/g beträgt.

20. Multimetalloxidmasse gemäß einer der Ausführungsformen 1 bis 19, deren Porengesamtvolumen 0,1 bis 0,8 cm$^3$/g beträgt.

21. Multimetalloxidmasse gemäß einer der Ausführungsformen 1 bis 20, deren Porengesamtvolumen 0,1 bis 0,5 cm$^3$/g beträgt.

22. Multimetalloxidmasse gemäß einer der Ausführungsformen 1 bis 21, deren Porengesamtvolumen 0,2 bis 0,4 cm$^3$/g beträgt.

23. Multimetalloxidmasse gemäß einer der Ausführungsformen 1 bis 22, bei der der Beitrag von Poren mit einem Porenradius $\leq$ 0,1 $\mu$m zum Porengesamtvolumen $\leq$ 0,05 cm$^3$/g beträgt.

24. Multimetalloxidmasse gemäß einer der Ausführungsformen 1 bis 23, bei der der Beitrag von Poren mit einem Porenradius im Bereich von 0,2 bis 0,4 $\mu$lm zum Porengesamtvolumen, bezogen auf das Porengesamtvolumen, $\geq$ 70 Vol.-% beträgt.

25. Multimetalloxidmasse gemäß einer der Ausführungsformen 1 bis 24, bei der der Beitrag von Poren mit einem Porenradius im Bereich von 0,2 bis 0,4 $\mu$m zum Porengesamtvolumen, bezogen auf das Porengesamtvolumen, $\geq$ 75 Vol.-% beträgt.

26. Multimetalloxidmasse gemäß einer der Ausführungsformen 1 bis 25, bei der der Beitrag von Poren mit einem Porenradius im Bereich von 0,2 bis 0,4 $\mu$m zum Porengesamtvolumen, bezogen auf das Porengesamtvolumen, $\geq$ 85 Vol.-% beträgt.

27. Multimetalloxidmasse gemäß einer der Ausführungsformen 1 bis 26, bei der der Beitrag von Poren mit einem Porenradius im Bereich von 0,2 bis 0,4 $\mu$m zum Porengesamtvolumen, bezogen auf das Porengesamtvolumen, $\geq$ 90 Vol.-% beträgt.

28. Multimetalloxidmasse gemäß einer der Ausführungsformen 1 bis 27, bei der der Beitrag von Poren mit einem Porenradius im Bereich von 0,2 bis 0,4 $\mu$m zum Porengesamtvolumen, bezogen auf das Porengesamtvolumen, $\geq$ 95 Vol.-% beträgt.

29. Multimetalloxidmasse gemäß einer der Ausführungsformen 1 bis 28, bei der bei einer Auftragung ihrer Porendurchmesser in $\mu$m auf der Abszisse und des Logarithmus des differentiellen Beitrags in cm$^3$/g des jeweiligen Porendurchmessers zum Porengesamtvolumen in cm$^3$/g auf der Ordinate eine monomodale Verteilungskurve resultiert.

30. Schalenkatalysator, umfassend einen Trägerformkörper und eine auf der äußeren Oberfläche des Trägerformkörpers befindliche Schale aus wenigstens einer Multimetalloxidmasse gemäß einer der Ausführungsformen 1 bis 29.

31. Schalenkatalysator gemäß Ausführungsform 30, bei dem die Schale aus der wenigstens einen Multimetalloxidmasse eine Dicke von 10 bis 1000 $\mu$m aufweist.

32. Schalenkatalysator gemäß Ausführungsform 30 oder 31, bei dem die Schale aus der wenigstens einen Multimetalloxidmasse eine Dicke von 100 bis 700 $\mu$m aufweist.

33. Schalenkatalysator gemäß einer der Ausführungsformen 30 bis 32, bei dem die Schale aus der wenigstens einen Multimetalloxidmasse eine Dicke von 300 bis 500 $\mu$m aufweist.

34. Schalenkatalysator gemäß einer der Ausführungsformen 30 bis 33, dessen Trägerformkörper eine Kugel, ein Vollzylinder oder ein Hohlzylinder ist.

35. Schalenkatalysator gemäß Ausführungsform 34, dessen Trägerformkörper eine Kugel ist, deren Durchmesser 1 bis 8 mm beträgt.

36. Schalenkatalysator gemäß Ausführungsform 34, dessen Trägerformkörper ein Zylinder ist, dessen Länge 2 bis 10 mm und dessen Außendurchmesser 4 bis 10 mm beträgt.

37. Schalenkatalysator gemäß Ausführungsform 34, dessen Trägerformkörper ein Ring mit einer Wanddicke von 1 bis 4 mm, einer Länge von 2 bis 10 mm und einem Außendurchmesser von 4 bis 10 mm ist.

38. Schalenkatalysator gemäß Ausführungsform 37, dessen Trägerformkörper ein Ring mit einer Wanddicke von 1 bis 2 mm, einer Länge von 3 bis 6 mm und einem Außendurchmesser von 4 bis 8 mm ist.

39. Schalenkatalysator gemäß Ausführungsform 37, dessen Trägerformkörper ein Ring mit einer Wanddicke von 1 bis 2 mm, einer Länge von 2 bis 4 mm und einem Außendurchmesser von 4 bis 8 mm ist.

40. Schalenkatalysator gemäß einer der Ausführungsformen 30 bis 39, wobei das Material des Trägerformkörpers Aluminiumoxid, Siliciumdioxid, ein Silikat, Siliciumcarbid, Zirkondioxid, Thoriumdioxid oder Steatit ist.

41. Vollkatalysatorformkörper, dessen Aktivmasse wenigstens ein Multimetalloxid gemäß einer der Ausführungsformen 1 bis 29 ist.

42. Vollkatalysatorformkörper gemäß Ausführungsform 41, der die Geometrie einer Kugel, eines Zylinders oder eines Ringes aufweist.

43. Vollkatalysatorformkörper gemäß Ausführungsform 42, der die Geometrie einer Kugel mit einem Durchmesser von 2 bis 10 mm aufweist.

44. Vollkatalysatorformkörper gemäß Ausführungsform 43, dessen Kugeldurchmesser 4 bis 8 mm beträgt.

45. Vollkatalysatorformkörper gemäß Ausführungsform 42, der die Geometrie eines Zylinders mit einer Länge von 2 bis 10 mm und einem Außendurchmesser von 2 bis 10 mm aufweist.

46. Vollkatalysatorformkörper gemäß Ausführungsform 45, wobei die Länge 2 bis 8 mm und der Außendurchmesser 2 bis 8 mm beträgt.

47. Vollkatalysatorformkörper gemäß Ausführungsform 42, der die Geometrie eines Ringes mit einer Wanddicke von 1 bis 3 mm, einer Länge von 2 bis 10 mm und einem Außendurchmesser von 2 bis 10 mm aufweist.

48. Vollkatalysatorformkörper gemäß Ausführungsform 47, dessen Länge 2 bis 8 mm und dessen Außendurchmesser 3 bis 8 mm beträgt.

49. Vollkatalysatorformkörper gemäß Ausführungsform 47, dessen Länge 3 bis 8 mm und dessen Außendurchmesser 3 bis 8 mm beträgt.

50. Vollkatalysatorformkörper gemäß einer der Ausführungsformen 47 bis 49, bei dem das Verhältnis von Länge zu Außendurchmesser 0,3 bis 0,7 beträgt.

51. Vollkatalysatorformkörper gemäß Ausführungsform 50, bei dem das Verhältnis von Länge zu Außendurchmesser 0,4 bis 0,6 beträgt.

52. Vollkatalysatorformkörper gemäß einer der Ausführungsformen 47 bis 51, bei dem das Verhältnis von Innendurchmesser zu Außendurchmesser 0,3 bis 0,7 beträgt.

53. Vollkatalysatorformkörper gemäß Ausführungsform 52, bei dem das Verhältnis von Innendurchmesser zu Außendurchmesser 0,4 bis 0,7 beträgt.

54. Vollkatalysatorformkörper gemäß Ausführungsform 47, dessen Ringgeometrie mit Außendurchmesser x Länge x Innendurchmesser eine Ringgeometrie aus der Gruppe 5 mm x 2 mm x 2mm, 5 mm x 3 mm x 2 mm, 5 mm x 3 mm x 3 mm, 5,5 mm x 3 mm x 3,5 mm, 6 mm x 3 mm x 4 mm, 6,5 mm x 3 mm x 4,5 mm, 7 mm x 3 mm x 5 mm, 7 mm x 7 mm x 3 mm, 7 mm x 3 mm x 4 mm, und 7 mm x 7 mm x 4 mm ist.

55. Verfahren zur Herstellung einer Multimetalloxidmasse gemäß einer der Ausführungsformen 1 bis 29, dadurch gekennzeichnet, dass man von Quellen ihrer elementaren Konstituenten ein feinteiliges inniges Trockengemisch erzeugt und dieses bei Temperaturen im Bereich von 350 bis 650 °C kalciniert.

56. Verfahren gemäß Ausführungsform 55, dadurch gekennzeichnet, dass die Kalcination unter Inertgas, unter einem Gemisch aus molekularem Sauerstoff und einem Inertgas, unter einer reduzierenden Atmosphäre oder unter Vakuum erfolgt.

57. Verfahren gemäß Ausführungsform 56, dadurch gekennzeichnet, dass die Kalcination unter Luft erfolgt.

58. Verfahren gemäß einer der Ausführungsformen 55 bis 57, dadurch gekennzeichnet, dass die Quellen in Form von Lösungen und/oder Suspensionen miteinander vermischt und die dabei resultierende nasse Mischung M zum feinteiligen innigen Trockengemisch getrocknet wird.

59. Verfahren gemäß Ausführungsform 58, dadurch gekennzeichnet, dass das Lösungs- und/oder Suspendiermittel eine wässrige Lösung ist.

60. Verfahren gemäß Ausführungsform 58 oder 59, dadurch gekennzeichnet, dass als Quellen nur Lösungen und/oder kolloidale Lösungen verwendet werden.

61. Verfahren gemäß Ausführungsform 60, dadurch gekennzeichnet, dass eine Quelle eine wässrige Lösung A ist, die Ausgangsverbindungen der Elemente Co, Fe und Bi gelöst enthält und deren pH-Wert $\leq 3$ und $\geq -2$ beträgt.

62. Verfahren gemäß Ausführungsform 60 oder 61, dadurch gekennzeichnet, dass eine Quelle eine wässrige Lösung B ist, die Ausgangsverbindungen der Elemente K und Mo gelöst enthält und deren pH-Wert $\leq 6,5$ und $\geq 3$ ist.

63. Verfahren gemäß einer der Ausführungsformen 60 bis 62, dadurch gekennzeichnet, dass eine Quelle wässriges Kieselsol ist.

64. Verfahren gemäß einer der Ausführungsformen 55 bis 63, dadurch gekennzeichnet, dass im Rahmen der Herstellung des feinteiligen innigen Trockengemischs eine wässrige Lösung A, die Ausgangsverbindungen der Elemente Co, Fe und Bi gelöst enthält und deren pH-Wert $\leq 3$ und $\geq -2$ beträgt, mit einer wässrigen Lösung B, die Ausgangsverbindungen der Elemente K und Mo gelöst enthält und deren pH-Wert $\leq 6,5$ und $\geq 3$ ist, vermischt wird und in das resultierende wässrige Gemisch ein wässriges Kieselsol eingemischt wird, wobei eine wässrige Mischung M resultiert.

65. Verfahren gemäß Ausführungsform 64, dadurch gekennzeichnet, dass die wässrige Lösung A in die wässrige Lösung B eingerührt wird.

66. Verfahren gemäß Ausführungsform 65, dadurch gekennzeichnet, dass das Einrühren bei einer Temperatur $\leq$ 80 °C und $\geq$ 0 °C erfolgt.

67. Verfahren gemäß Ausführungsform 66, dadurch gekennzeichnet, dass das Einrühren bei einer Temperatur $\leq$ 70 °C und $\geq$ 0 °C erfolgt.

68. Verfahren gemäß Ausführungsform 66 oder 67, dadurch gekennzeichnet, dass das Einrühren bei einer Temperatur $\leq$ 60 °C und $\geq$ 0 °C erfolgt.

69. Verfahren gemäß einer der Ausführungsformen 66 bis 68, dadurch gekennzeichnet, dass das Einrühren bei einer Temperatur $\leq$ 40 °C und $\geq$ 0 °C erfolgt.

70. Verfahren gemäß einer der Ausführungsformen 64 bis 69, dadurch gekennzeichnet, dass das Verhältnis V, gebildet aus der in der wässrigen Mischung der wässrigen Lösung A und der wässrigen Lösung B wahlweise

enthaltenen molaren Gesamtmenge $n_1$ an $NH_3$ und $NH_4^+$ und der in derselben wässrigen Mischung enthaltenen molaren Gesamtmenge $n_2$ an Mo, $V = n_1 : n_2, \leq 1$ beträgt.

71. Verfahren gemäß Ausführungsform 70, dadurch gekennzeichnet, dass $0 \leq V \leq 6/7$ ist.

72. Verfahren gemäß einer der Ausführungsformen 64 bis 71, dadurch gekennzeichnet, dass der pH-Wert der wässrigen Mischung aus der wässrigen Lösung A und der wässrigen Lösung B $\leq 3$ und $\geq 0$ beträgt.

73. Verfahren gemäß einer der Ausführungsformen 64 bis 72, dadurch gekennzeichnet, dass der $SiO_2$-Gehalt des wässrigen Kieselsols 15 bis 60 Gew.-% beträgt.

74. Verfahren gemäß einer der Ausführungsformen 64 bis 73, dadurch gekennzeichnet, dass der $SiO_2$-Gehalt des wässrigen Kieselsols 30 bis 60 Gew.-% beträgt.

75. Verfahren gemäß einer der Ausführungsformen 64 bis 74, dadurch gekennzeichnet, dass der $SiO_2$-Gehalt des wässrigen Kieselsols 45 bis 55 Gew.-% beträgt

76. Verfahren gemäß einer der Ausführungsformen 64 bis 75, dadurch gekennzeichnet, dass der Gesamtgehalt der wässrigen Mischung M an Mo, Co, Fe, Bi und Si, bezogen auf die Menge an in der wässrigen Mischung M enthaltenem Wasser, 5 bis 25 Gew.-% beträgt.

77. Verfahren gemäß Ausführungsform 76, dadurch gekennzeichnet, dass der Gesamtgehalt der wässrigen Mischung M an Mo, Co, Fe, Bi und Si, bezogen auf die Menge an in der wässrigen Mischung M enthaltenem Wasser, 8 bis 20 Gew.-% beträgt.

78. Verfahren gemäß einer der Ausführungsformen 64 bis 77, dadurch gekennzeichnet, dass der pH-Wert der wässrigen Mischung M $\leq 3$ beträgt.

79. Verfahren gemäß Ausführungsform 78, dadurch gekennzeichnet, dass der pH-Wert der wässrigen Mischung M $\geq 0$ und $\leq 2$ beträgt.

80. Verfahren gemäß einer der Ausführungsformen 64 bis 79, dadurch gekennzeichnet, dass der im wässrigen Medium der wässrigen Mischung M gelöst vorliegende Anteil AT an Co der in der wässrigen Mischung M insgesamt enthaltenen Menge an Co $\leq 60$ % beträgt.

81. Verfahren gemäß Ausführungsform 80, dadurch gekennzeichnet, dass AT $\leq 50$ % beträgt.

82. Verfahren gemäß Ausführungsform 80 oder 81, dadurch gekennzeichnet, dass 15 % $\leq$ AT $\leq 40$ % gilt.

83. Verfahren gemäß einer der Ausführungsformen 58 bis 82, dadurch gekennzeichnet, dass die Trocknung durch Sprühtrocknung erfolgt.

84. Verfahren gemäß Ausführungsform 83, dadurch gekennzeichnet, dass die Sprühtrocknung der wässrigen Mischung M nach Versprühen derselben in einem heißem Gasstrom erfolgt, dessen Eintrittstemperatur 250 bis 450 °C beträgt.

85. Verfahren gemäß einer der Ausführungsformen 55 bis 84, dadurch gekennzeichnet, dass man das innige Trockengemisch vorab der Kalcinierung, wahlweise unter Zusatz von Formgebungshilfsmitteln, zu Formkörpern von regelmäßiger oder unregelmäßiger Geometrie formt.

86. Verfahren gemäß Ausführungsform 85, dadurch gekennzeichnet, dass die Formgebung durch Tablettieren erfolgt.

87. Verfahren gemäß Ausführungsform 85 oder 86, dadurch gekennzeichnet, dass als Formgebungshilfsmittel Graphit zugesetzt wird.

88. Verfahren gemäß einer der Ausführungsformen 85 bis 87, dadurch gekennzeichnet, dass der Formkörper die Geometrie eines Ringes aufweist.

89. Verfahren gemäß Ausführungsform 88, dadurch gekennzeichnet, dass der Ring einen Außendurchmesser von 2 bis 10 mm, eine Höhe von 2 bis 10 mm und eine Wandstärke von 1 bis 3 mm aufweist.

90. Verfahren gemäß Ausführungsform 89, dadurch gekennzeichnet, dass der Ring einen Außendurchmesser von 2 bis 8 mm und eine Höhe von 2 bis 8 mm aufweist.

91. Verfahren gemäß Ausführungsform 89 oder 90, dadurch gekennzeichnet, dass der Ring einen Außendurchmesser von 3 bis 8 mm und eine Höhe von 3 bis 8 mm aufweist.

92. Verfahren gemäß einer der Ausführungsformen 88 bis 91, dadurch gekennzeichnet, dass die Seitendruckfestigkeit SD des ringförmigen Formkörpers die Relation 12 N ≤ SD ≤ 35 N erfüllt.

93. Verfahren gemäß Ausführungsform 92, dadurch gekennzeichnet, dass die Seitendruckfestigkeit die Relation 15 N ≤ SD ≤ 30 N erfüllt.

94. Verfahren gemäß Ausführungsform 92 oder 93, dadurch gekennzeichnet, dass die Seitendruckfestigkeit die Relation 19 N ≤ SD ≤ 30 N erfüllt.

95. Verfahren gemäß einer der Ausführungsformen 55 bis 94, dadurch gekennzeichnet, dass bei der Kalcination die Temperatur von 600 °C nicht überschritten wird.

96. Verfahren gemäß einer der Ausführungsformen 55 bis 95, dadurch gekennzeichnet, dass bei der Kalcination die Temperatur von 550 °C nicht überschritten wird.

97. Verfahren gemäß einer der Ausführungsformen 55 bis 96, dadurch gekennzeichnet, dass bei der Kalcination die Temperatur von 500 °C nicht überschritten wird.

98. Verfahren gemäß einer der Ausführungsformen 55 bis 97, dadurch gekennzeichnet, dass vorab der Kalcination eine thermische Behandlung des innigen Trockengemischs bei Temperaturen ≥ 120 °C und ≤ 350 °C erfolgt.

99. Verfahren gemäß einer der Ausführungsformen 55 bis 98, dadurch gekennzeichnet, dass vorab der Kalcination eine thermische Behandlung des innigen Trockengemischs bei Temperaturen ≥ 150 °C und ≤ 320 °C erfolgt.

100. Verfahren gemäß einer der Ausführungsformen 55 bis 99, dadurch gekennzeichnet, dass vorab der Kalcination eine thermische Behandlung des innigen Trockengemischs bei Temperaturen ≥ 220 °C und ≤ 290 °C erfolgt.

101. Verfahren der heterogen katalysierten partiellen Gasphasenoxidation eines 3 bis 6 C-Atome aufweisenden Alkans, Alkanols, Alkanals, Alkens und/oder Alkenals an einem Katalysatorbett, dadurch gekennzeichnet, dass das Katalysatorbett wenigstens eine Multimetalloxidmasse gemäß einer der Ausführungsformen 1 bis 29 aufweist.

102. Verfahren der heterogen katalysierten partiellen Gasphasenoxidation eines 3 bis 6 C-Atome aufweisenden Alkans, Alkanols, Alkanals, Alkens und/oder Alkenals an einem Katalysatorbett, dadurch gekennzeichnet, dass das Katalysatorbett wenigstens einen Katalysator gemäß einer der Ausführungsformen 30 bis 54 aufweist.

103. Verfahren der heterogen katalysierten partiellen Gasphasenoxidation eines 3 bis 6 C-Atome aufweisenden Alkans, Alkanols, Alkanals, Alkens und/oder Alkenals an einem Katalysatorbett, dadurch gekennzeichnet, dass das Katalysatorbett wenigstens ein Produkt eines Verfahrens gemäß einer der Ausführungsformen 55 bis 100 aufweist.

104. Verfahren gemäß einer der Ausführungsformen 101 bis 103, dadurch gekennzeichnet, dass es ein Verfahren der heterogen katalysierten partiellen Gasphasenoxidation von Propen zu Acrolein oder von iso-Buten zu Methacrolein ist.

105. Verfahren gemäß einer der Ausführungsformen 101 bis 103, dadurch gekennzeichnet, dass es ein Verfahren der Ammoxidation von Propen zu Acrylnitril oder ein Verfahren der Ammoxidation von iso-Buten zu Methacrylnitril ist.

106. Verwendung wenigstens eines Multimetalloxids gemäß einer der Ausführungsformen 1 bis 29, oder wenigstens eines Katalysators gemäß einer der Ausführungsformen 30 bis 54, oder wenigstens eines Produkts eines Verfahrens gemäß einer der Ausführungsformen 55 bis 100 zur Katalyse eines Verfahrens der heterogen katalysierten partiellen Gasphasenoxidation eines 3 bis 6 C-Atome aufweisenden Alkans, Alkanols, Alkanals, Alkens und/oder Alkenals an einem Katalysatorbett.

Beispiele und Vergleichsbeispiele

I. Angewandtes Verfahren zur Herstellung von ringförmigen Vollkatalysatorformkörpern B1 bis B4 sowie V1 bis V7

1. Herstellung der jeweiligen wässrigen Lösung B

In einem zur Atmosphäre (1 atm, 1,01 bar) offenen, temperierbaren und mit einem Ankerrührer ausgestatteten Behälter aus Edelstahl (Innenvolumen = 10 dm$^3$) wurde die in der Tabelle 1 jeweils aufgeführte Menge M1 an vollentsalztem Wasser vorgelegt und unter Rühren (150 U/min) auf 60 °C erhitzt. Anschließend wurde jeweils die in Tabelle 1 aufgeführte Menge M2 einer 32 gew.-%igen wässrigen Lösung von KOH in Wasser, die eine Temperatur von 60 °C aufwies, zugegeben. Nach 1-minütigem Nachrühren bei 60 °C wurde unter Beibehalt der 60 °C jeweils die in Tabelle 1 aufgeführte Menge M3 an Ammoniumheptamolybdattetrahydrat (weiße Kristalle mit einer Körnung d < 1 mm, 55 Gew.-% Mo, 7,0 - 8,5 Gew.-% NH$_3$, max. 150 mg/kg Alkalimetalle, der Fa. H.C. Starck, D-38642 Goslar) portionsweise eingerührt und die dabei resultierende wässrige Lösung bei 60 °C 20 min nachgerührt (150 U/min). Daran anschließend wurde die in Tabelle 1 aufgeführte Menge M4 an Ammoniumparawolframat (71 Gew.-%, H.C. Starck, D-38642 Goslar) zugegeben und weitere 20 min bei 60 °C und 150 U/min gerührt. Der pH-Wert der dabei jeweils resultierenden wässrigen Lösung B lag im Bereich 4 bis 6.

2. Herstellung der jeweiligen wässrigen Lösung A

In einem zur Atmosphäre (1 atm, 1,01 bar) offenen, temperierbaren und mit einem Ankerrührer ausgestatteten Behälter aus Edelstahl (Innenvolumen = 5 dm$^3$) wurde jeweils die in der Tabelle 1 aufgeführte Menge M5 einer wässrigen Kobalt-(II)-nitratlösung (12,4 Gew.-% Co, 27 Gew.-% Nitrat (NO$_3^-$)), pH = 1, hergestellt durch Lösen von Kobaltmetall der Fa. MFT Metals & Ferro-Alloys Trading GmbH, D-41474 Viersen, Reinheit > 99,6 Gew.-% Co, < 0,3 Gew.-% Ni, < 100 mg/kg Fe, < 50 mg/kg Cu in Salpetersäure) vorgelegt und unter Rühren (150 U/min) auf 60 °C erwärmt. Unter fortgesetztem Rühren (150 U/min) und fortgesetzter Temperierung auf 60 °C wurde jeweils die in Tabelle 1 aufgeführte Menge M6 an kristallinem Eisen-(III)-nitratnonahydrat (13,6 Gew.-% Fe, < 0,4 Gew.-% Alkalimetalle, < 0,01 Gew.-% Chlorid, < 0,02 Gew.-% Sulfat, der Fa. Dr. Paul Lohmann GmbH, D-81857 Emmerthal) zudosiert und 10 min bei 60 °C nachgerührt. Zur dabei resultierenden wässrigen Lösung wurde jeweils die in Tabelle 1 aufgeführte Menge M7 einer 60 °C warmen, wässrigen, salpetersauren Bismutnitratlösung (10,8 Gew.-% Bi, 13 Gew.-% Nitrat, hergestellt durch Lösen von Bismutmetall der Fa. Sidech S.A., BE-1495 Tilly, Reinheit > 99,997 Gew.-% Bi, < 7 mg/kg Pb, je < 5 mg/kg Ni, Ag, Fe, je < 3 mg/kg Cu, Sb und < 1 mg/kg Cd, Zn in Salpetersäure) gegeben und 10 min bei 60 °C nachgerührt. Der pH-Wert der jeweils resultierenden wässrigen Lösung A lag im Bereich von -1 bis 0.

3. Vermischen der jeweiligen wässrigen Lösung A mit der jeweiligen wässrigen Lösung B

Die jeweilige 60 °C warme wässrige Lösung A wurde mit Hilfe einer Schlauchpumpe (Typ: BVP, Firma: Ismatec SA, Labortechnik-Analytik, Feldeggstraße 6, CH-8152 Glattbrugg, Einstellung: 320 Skalenteile) innerhalb von 15 min kontinuierlich in die nun mit einem Ultra-Turrax (Firma Janke & Kunkel GmbH & Co.KG - IKA-Labortechnik, Janke & Kunkel-Str. 10, DE-79219 Staufen, Schaft-Typ: 550KR-G45 fein, Schaftrohrdurchmesser: 25 mm, Statordurchmesser: 45 mm, Rotordurchmesser: 40 mm, Einstellung: Stufe 5) intensiv gerührte 60 °C warme jeweilige wässrige Lösung B eindosiert. Die Aufgabe der wässrigen Lösung A erfolgte dabei auf der Höhe des Rotors des Ultra-Turax Rührers um etwa 0,5 bis 1 cm von der Außenkante des Rotors des Ultra-Turax Rührers versetzt. Die entstehende wässrige Suspension wurde noch 15 min bei 60 °C nachgerührt.

4. Zugabe eines Kieselgels unter Erhalt der jeweiligen wässrigen Mischung M

Anschließend wurde dem in "3." jeweils erhaltenen wässrigen Gemisch jeweils die in Tabelle 1 aufgeführte Menge M8 eines auf 60 °C erwärmten Kieselgels der Fa. Grace vom Typ Ludox TM50 (24,4 Gew.-% Si, Dichte: 1,29 g/cm$^3$, pH: 8,5 bis 9,5, Alkaligehalt max. 0,5 Gew.-%) zugegeben und daran anschließend noch weitere 15 min bei 60 °C gerührt. Der pH-Wert der jeweils resultierenden wässrigen Mischung M lag im Bereich von 1 bis 2 (im Fall der Vollkatalysatorformkörper V1, V2, V3, V4, V6 und V7 sowie B1 und B3) bzw. im Bereich von 0 bis 1 (im Fall der Vollkatalysatorformkörper B2, B4 und V5).

Der Feststoffgehalt der verschiedenen erhaltenen wässrigen Mischungen M (der Begriff Feststoff subsumiert dabei die nicht zusätzlich getrocknete Masse, die sich beim nachfolgend beschriebenen Zentrifugieren als festes Sediment abscheidet: Zentrifuge Universal 16 der Fa. Hettich, Drehzahl: 3000 U/min, Zentrifugationsdauer: 10 min, Verwendung von Zentrifugengläsern mit 100 ml Füllvolumen, Bezugstemperatur: 60 °C) lag, bezogen auf das Gewicht der jeweiligen wässrigen Mischung M, im Bereich von 20 bis 40 Gew.-% (im Fall der Vollkatalysatorformkörper V1, V2, V3, V4, V6 und V7 sowie B1 und B3) bzw. im Bereich von 40 bis 60 Gew.-% (im Fall der Vollkatalysatorformkörper B2, B4 und V5). Der flüssige Überstand enthielt, bezogen auf die in der jeweiligen wässrigen Mischung M enthaltene Gesamtmenge an Kobalt, 60 bis 90 Gew.-% an Co gelöst (im Fall der Vollkatalysatorformkörper V1, V2, V3, V4, V6 und V7 sowie B1 und B3) bzw. 40 bis 60 Gew.-% an Co gelöst (im Fall der Vollkatalysatorformkörper B2, B4 und V5). Die Trennung von Sediment und Überstand erfolgte jeweils durch Dekantieren.

5. Sprühtrocknung der jeweiligen wässrigen Mischung M

Die jeweils (auch während der Sprühtrocknung) bei 60 °C mittels des Ankerrührers (150 U/min) weiter gerührte jeweilige wässrige Mischung (Suspension) M wurde in einem Sprühturm vom Typ Mobile Minor™ 2000 (MM-I) der Firma Niro A/S, Gladsaxevej 305, 2860 Søborg, Dänemark, mit einem Zentrifugalzerstäuber vom Typ F01A und einem Zerstäuberrad vom Typ SL24-50 im Heißluftgleichstrom innerhalb von 90 bis 140 min sprühgetrocknet (Gaseintrittstemperatur: 350 $\pm$ 10 °C, Gasaustrittstemperatur: 140 $\pm$ 5 °C). Der noch nicht sprühgetrocknete Anteil wurde stets bei 60 °C weitergerührt. Die Einstellung der Drehzahl des Zerstäuberrads betrug 25000 U/min. Es wurden so jeweils etwa 700 g (im Fall der Vollkatalysatorformkörper V1, V2, V3, V4, V6 und V7 sowie B1 und B3) bzw. etwa 1100 g (im Fall der Vollkatalysatorformkörper B2, B4 und V5) an orange-braunem Sprühpulver erhalten. Die Restfeuchten der verschiedenen Sprühpulver (Restfeuchtebestimmung mittels Mikrowellen) lagen im Bereich von 3 (im Fall des Vollkatalysatorkörpers B3) bis 7,7 Gew.-% (jeweils bezogen auf das Gesamtgewicht des jeweiligen Sprühpulvers). Der Glühverlust der verschiedenen Sprühpulver (3 h bei 600 °C (Pulvertemperatur) unter stehender Luft glühen) war in allen Fällen < 35 Gew.-%.

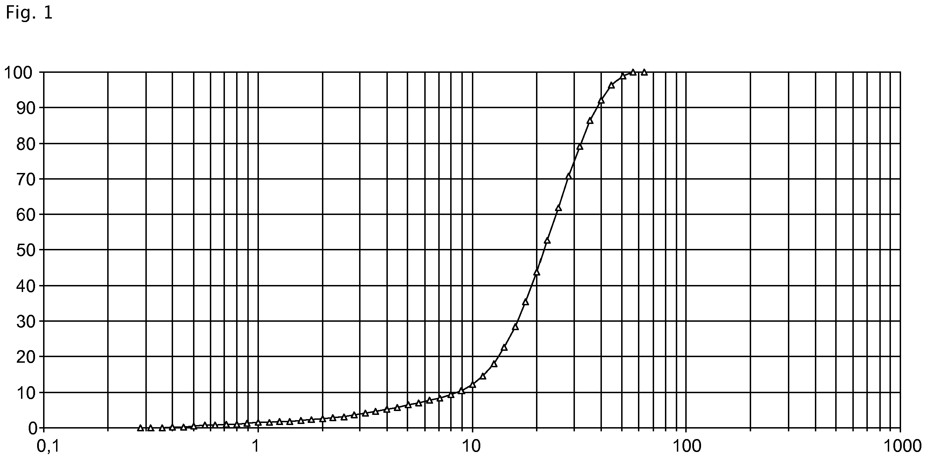

Eine repräsentative Sprühpulverpartikeldurchmesserverteilung (Dispergierdruck = 2 bar absolut) mit $d_{10}$ = 9 $\mu$m, $d_{50}$ = 22 $\mu$m und $d_{90}$ = 39 $\mu$m zeigt Figur 1 (für den Fall des Vollkatalysatorformkörpers B3). Die Abszisse zeigt dabei den jeweiligen Partikeldurchmesser ($\mu$m) im logarithmischen Maßstab und die zugehörige Ordinate zeigt den prozentualen Volumenanteil des Sprühpulvers (bezogen auf dessen Gesamtvolumen) der aus Partikeln des jeweiligen Durchmessers und aus Partikeln eines kleineren Durchmessers besteht (Vol.-%).

Tabelle 1

| Vollkatalysatorformkörper | in der wässrigen Mischung M vorliegende Einwaagestöchiometrie | M1 [g] | M2 [g] | M3 [g] | M4 [g] | M5 [g] | M6 [g] | M7 [g] | M8 [g] |
|---|---|---|---|---|---|---|---|---|---|
| B1 | $Bi_{0,6} Mo_{12} Co_{8,0} Fe_{2,0} Si_{1,6} K_{0,08}$ | 4750 | 2,65 | 568,53 | 0 | 1032,68 | 223,06 | 315,33 | 50,2 |
| B2 | $Bi_{0,6} Mo_{12} Co_{8,0} Fe_{2,0} Si_{1,6} K_{0,08}$ | 2375 | 3,98 | 852,80 | 0 | 1549,02 | 334,60 | 473,00 | 75,03 |
| B3 | $Bi_{0,6} Mo_{12} Co_{8,4} Fe_{2,07} Si_{1,6} K_{0,08}$ | 4750 | 2,65 | 568,53 | 0 | 1084,31 | 230,87 | 315,33 | 50,02 |
| B4 | $Bi_{0,6} Mo_{12} Co_{8,4} Fe_{2,07} Si_{1,6} K_{0,08}$ | 2375 | 3,98 | 852,80 | 0 | 1626,47 | 346,31 | 473,00 | 75,03 |
| V1 | $Bi_{0,6} Mo_{12} Co_{8,7} Fe_{2,3} Si_{1,6} K_{0,08}$ | 4750 | 2,65 | 568,53 | 0 | 1123,04 | 256,52 | 315,33 | 50,02 |
| V2 | $Bi_{0,1} Mo_{12} Co_{8,0} Fe_{2,0} Si_{1,6} K_{0,08}$ | 4750 | 2,65 | 568,53 | 0 | 1032,68 | 223,06 | 52,56 | 50,02 |
| V3 | $Bi_{2,0} Mo_{12} Co_{8,0} Fe_{2,0} Si_{1,6} K_{0,08}$ | 4750 | 2,65 | 568,53 | 0 | 1032,68 | 223,06 | 1051,11 | 50,02 |
| V4 | $Bi_{0,6} Mo_{12} Co_{7,0} Fe_{3,0} Si_{1,6} K_{0,08}$ | 4750 | 2,65 | 568,53 | 0 | 903,60 | 334,60 | 315,33 | 50,02 |
| V5 | $Bi_{0,6} Mo_{12} Co_{7,0} Fe_{3,0} Si_{1,6} K_{0,08}$ | 2375 | 3,98 | 852,80 | 0 | 1355,39 | 501,89 | 473,00 | 75,03 |
| V6 | $Bi_{0,6} Mo_{12} Co_{4,6} Fe_{4,6} Si_{1,6} K_{0,08}$ | 4750 | 2,65 | 568,53 | 0 | 593,79 | 513,05 | 315,33 | 50,02 |
| V7 | $Bi_{0,6} Mo_{12} Co_{8,4} Fe_{2,07} Si_{1,6} W_{0,5} K_{0,08}$ | 4750 | 2,65 | 568,53 | 35,17 | 1084,31 | 230,87 | 315,33 | 50,02 |

6. Herstellung ringförmiger Vollkatalysatorvorläuferformkörper (unter N$_2$-Atmosphäre)

In das jeweilige Sprühpulver wurden in einem Rhönradmischer (Raddurchmesser: 650 mm, Trommelvolumen: 5 l), bezogen auf sein Gewicht, 1 Gew.-% feinteiliger Graphit der Sorte TIMREX T44 der Fa. Timcal Ltd., CH-6743 Bodio (vgl. WO 2008/087116) homogen verteilt eingemischt (Drehzahl: 30 U/min, Einmischdauer: 30 min). In einem Laborkalander mit 2 gegenläufigen Stahlwalzen (Walzendurchmesser: 10 cm; zur Zwischenkompaktierung genutzte Walzenlänge: 13,5 cm; Walzendrehzahl: 10 U/min) wurde das jeweils resultierende homogene Gemisch bei einem Pressdruck von 9 bar kompaktiert und daran anschließend durch ein Sieb mit quadratischen Maschen (Kantenlänge = 0,8 mm) gedrückt. In das jeweilige, wie beschrieben vergröberte, Sprühpulver wurden im vorstehend bereits beschriebenen Rhönradmischer (30 U/min, 30 min Einmischdauer), bezogen auf sein Gewicht, weitere 2,5 Gew.-% desselben feinteiligen Graphits eingemischt. Anschließend wurde das erhaltene feinteilige innige Trockengemisch wie in der DE-A 10200804093 beschrieben, mit einem Kilian Rundläufer (9-fach-Tablettiermaschine) vom Typ S100 (Fa. Kilian, D-50735 Köln) unter Stickstoffatmosphäre und einer Umgebungstemperatur von 25 °C zu ringförmigen Vollkatalysatorvorläuferformkörpern der Geometrie A x H x I = 5 mm x 3 mm x 2 mm, einer Seitendruckfestigkeit im Bereich 19 N bis 30 N (im Fall des Vollkatalysatorformkörpers B3 = 30 N) und einer Masse von 119 ± 2 mg verdichtet (tablettiert). Die Presskraft betrug 3,0 bis 3,5 kN und die Füllhöhe war 7,5 bis 9 mm.

7. Thermische Vorbehandlung und Kalcination der jeweiligen ringförmigen Vollkatalysatorvorläuferformkörper

Zur abschließenden thermischen Behandlung wurden jeweils 1000 g der jeweils hergestellten Vollkatalysatorvorläuferformkörper gleichmäßig aufgeteilt auf nebeneinander angeordnete 4 Gitternetze mit einer quadratischen Grundfläche von jeweils 150 mm x 150 mm (Schütthöhe: 15 mm) in einem mit 4500 Nl/h zuvor getrockneter Luft (die eine Eintrittstemperatur von 140 °C aufwies) durchströmten Umluftschachtofen (Fa. Nabertherm; Ofen Modell S60/65A) aufgebracht (der Umluftofen befand sich in einer 25 °C aufweisenden Umgebung). Anschließend wurde unter Beibehalt des Luftstroms (einschließlich seiner Eintrittstemperatur) die Temperatur im Umluftschachtofen wie folgt variiert (die Temperaturangaben meinen die Temperatur im jeweiligen aufgebrachten Schüttgut; diese wurde mittels 4 Thermoelementen bestimmt, die sich jeweils in der geometrischen Mitte der 4 Gitternetze im Zentrum des auf dem jeweiligen Gitternetz aufgebrachten Schüttgutes befanden; eines der Thermoelemente stellte den Istwert zur Temperaturregelung des Umluftschachtofens bereit; die anderen Thermoelemente bestätigten, dass die Temperaturen innerhalb des Intervalls ± 0,1 °C identisch waren). Die Temperaturerhöhungen erfolgten über die Zeit im Wesentlichen linear. Innerhalb von 72 min wurde von 25 °C auf 130 °C aufgeheizt. Diese Temperatur wurde während 72 min aufrecht erhalten und dann innerhalb von 36 min auf 190 °C erhöht. Die 190 °C wurden 72 min gehalten, bevor die Temperatur innerhalb von 36 min auf 220 °C erhöht wurde. Die 220 °C wurden 72 min gehalten, bevor die Temperatur innerhalb von 36 min auf 265 °C erhöht wurde. Die 265 °C wurden 72 min gehalten, bevor die Temperatur innerhalb von 93 min auf 380 °C erhöht wurde. Die 380 °C wurden 187 min gehalten, bevor die Temperatur innerhalb von 93 min auf 430 °C erhöht wurde. Die 430 °C wurden 187 min gehalten, bevor innerhalb von 93 min auf die Endkalcinationstemperatur von 500 °C erhöht wurde. Diese wurde 467 min aufrecht erhalten. Dann wurde innerhalb von 12 h auf 25 °C abgekühlt. Hierzu wurde sowohl die Beheizung des Umluftschaftofens als auch die Luftstromvorheizung abgeschaltet (der Luftstrom von 4500 Nl/h als solcher wurde jedoch beibehalten; die Eintrittstemperatur des Luftstroms betrug dann 25 °C). Die spezifischen BET Oberflächen der erhaltenen ringförmigen Vollkatalysatorformkörper lagen im Bereich 4 bis 8 m$^2$/g (im Fall des ringförmigen Vollkatalysatorformkörpers B3 war die spezifische BET-Oberfläche 7,6 m$^2$/g). Der Graphitgehalt der erhaltenen ringförmigen Vollkatalysatorformkörper betrug noch 2,4 Gew.-% (bezogen auf ihr Gesamtgewicht). In entsprechender Weise weisen die durch Bandkalcination erhältlichen Vergleichskatalysatorformlörper VK1 - 1 der WO 2010/066645 noch einen Gehalt des in selbiger Schrift zu ihrer Herstellung verwendeten Graphits von 3,45 Gew.-% auf. Die ringähnlichen Multimetalloxid - Vollkatalysatoren II der DE 102009047291 A1 weisen analog noch einen Graphitgehalt von 3,8 Gew.-% auf. Wird zu ihrer Herstellung anstelle des Graphits Asbury 3160 der Graphit Timrex T44 der Firma Timcal AG in gleichen Gewichtsmengen eingesetzt, liegt der verbliebene Graphitgehalt nur noch bei 1,4 Gew.-%. Der Graphitgehalt der ringförmigen Vollkatalysatorformkörper aus dem Abschnitt B) der Beispiele und Vergleichsbeispiele der DE 102007004961 A1 liegt in allen Fällen im Bereich von 1 bis 4,5 Gew.-%. Wird in diesem Abschnitt B) bei der Herstellung der Vollkatalysatorformkörper als Graphit der Asbury 3160 verwendet, liegt der Graphitgehalt des resultierenden Vollkatalysatorformkörpers bei 3,8 Gew.-%. Wird in diesem Abschnitt B) als Graphit der Timrex 44 eingesetzt, beträgt der Graphitgehalt der resultierenden Vollkatalysatorformkörper 1,4 Gew.-%.

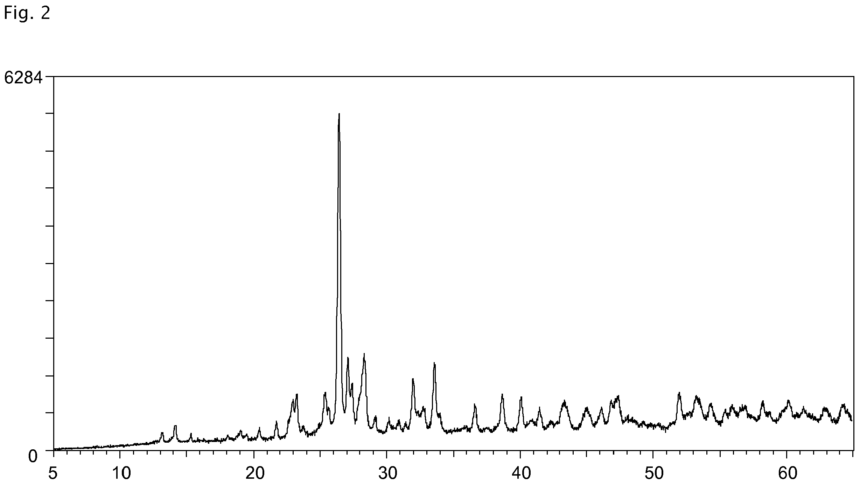

Figur 2 zeigt am Beispiel des Vollkatalysatorformkörpers B3 ein repräsentatives XRD Dlffraktogramm. Die Abszisse zeigt dabei den Beugungswinkel in der 2Θ-Schale (2 Theta-Skala) und die Ordinate zeigt die absolute Intensität der gebeugten Röntgenstrahlung.

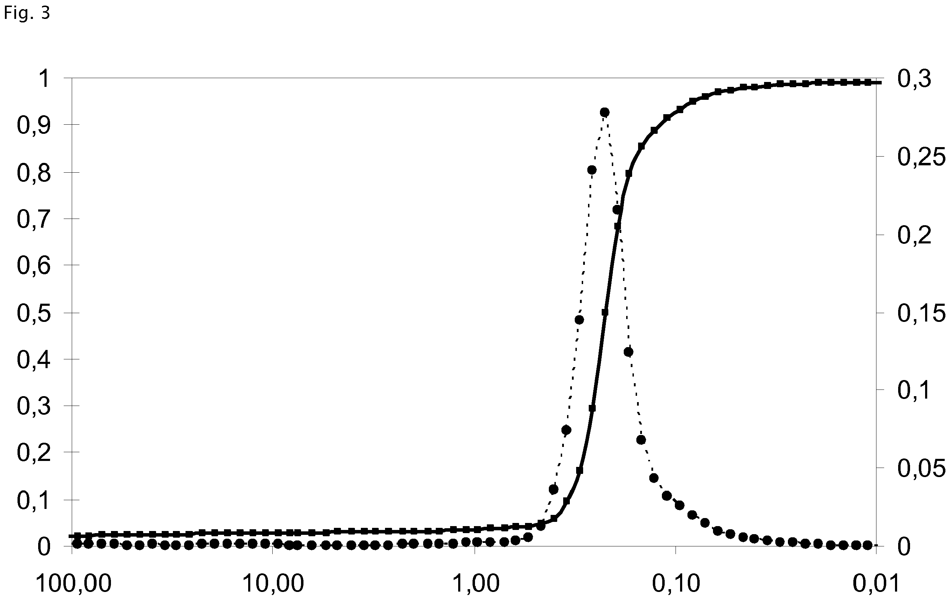

Figur 3 zeigt ebenfalls am Beispiel des Vollkatalysatorformkörpers B3 eine repräsentative Porenverteilung. Auf der Abszisse ist der Porendurchmesser in μm aufgetragen. Auf der linken Ordinate ist der Logarithmus des differentiellen Beitrags in cm$^3$/g des jeweiligen Porendurchmessers zum Porengesamtvolumen/g eingetragen. Das Maximum weist den Porendurchmesser mit dem größten Beitrag zum Porengesamtvolumen aus. Auf der rechten Ordinate ist in cm³/g das Integral über die individuellen Beiträge der einzelnen Porendurchmesser zum Porengesamtvolumen/g aufgetragen. Der Endpunkt ist das Porengesamtvolumen/g.

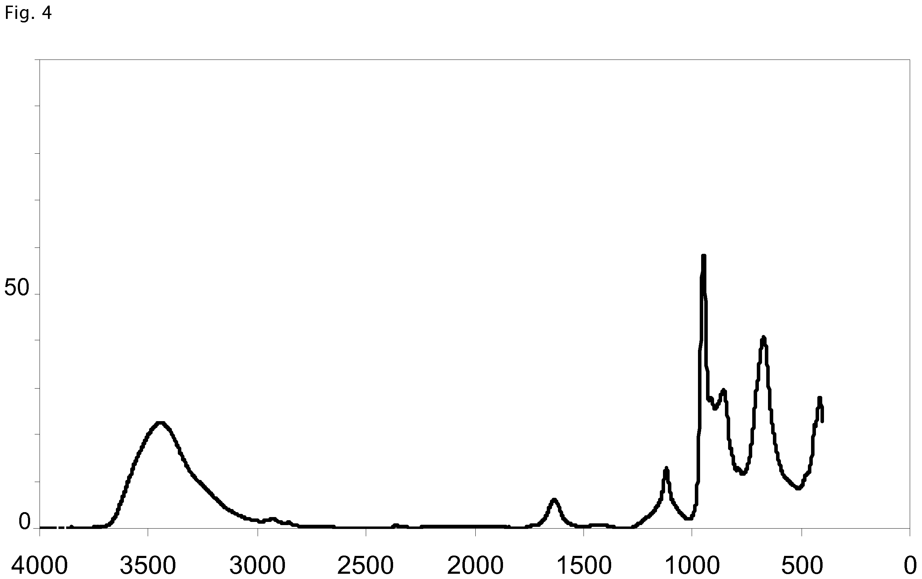

Figur 4 zeigt am Beispiel des Vollkatalysatorformkörpers B3 ein repräsentatives Infrarot-Transmissionsspektrum. Auf der Abszisse ist die Wellenzahl in cm⁻¹, auf der Ordinate der Transmissionsgrad in % aufgetragen. Als Messgerät wurde das FTIR (Fourier Transformiertes Infrarot)-Spektrometer Nicolet 6700 der Fa. Thermo Fisher Scientific verwendet. Die Messungen erfolgten, nach einer Verreibung der jeweiligen Vollkatalysatorformkörper mit Mörser und Pistill (auf Partikelgrößen < 0,1 mm), an anschließend aus der jeweiligen Verreibung unter Zugabe von feinteiligem KBr als IR-inaktivem Verdünnungsmaterial erzeugten Presslingen. Die zugehörigen Messparameter waren wie folgt: Auflösung = 4 cm⁻¹; Messbereich = 4000 bis 400 cm⁻¹; Anzahl Scans = 32; Messart = Transmission.

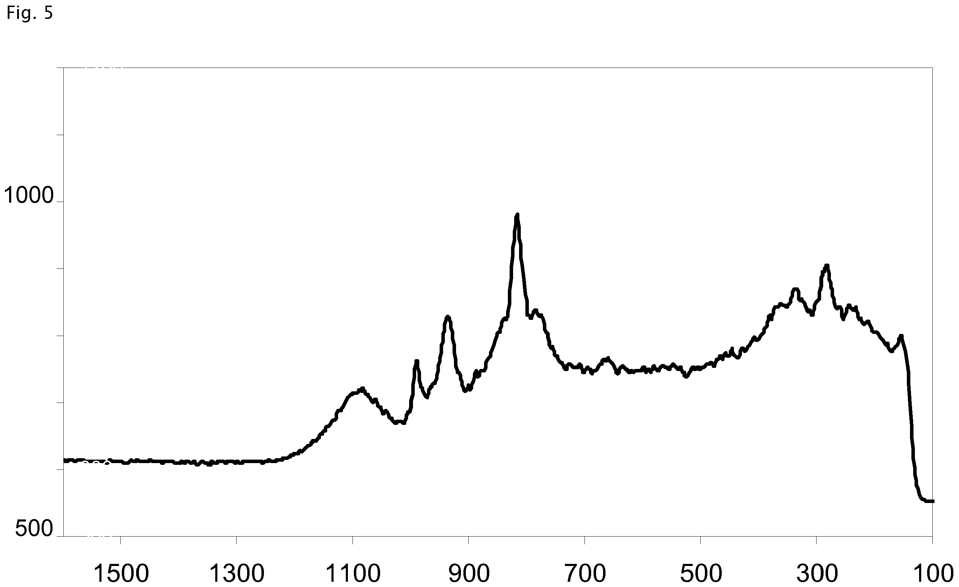

Figur 5 zeigt am Beispiel des Vollkatalysatorformkörpers B3 ein repräsentatives Raman-Spektrum. Auf der Abszisse ist die Raman-Verschiebung in cm⁻¹, auf der Ordinate die zugehörige Raman-Intensität aufgetragen. Als Messgerät wurde das Raman Mikroskop Alpha 300 R der Fa. WiTec eingesetzt. Die Messparameter bei der Raman-Spektroskopie waren folgende: Anregungswellenlänge des Lasers: 532 nm; Gitter: 600 grids/mm; Anzahl der Akkumulationen: 100; Integrationszeit: 0,2 sec; verwendetes Objektiv: das Makroset mit f = 30 mm. Die Messungen erfolgten jeweils an einer Verreibung der jeweiligen Vollkatalysatorformkörper auf eine Partikelgröße < 0,1 mm mit Mörser und Pistill.

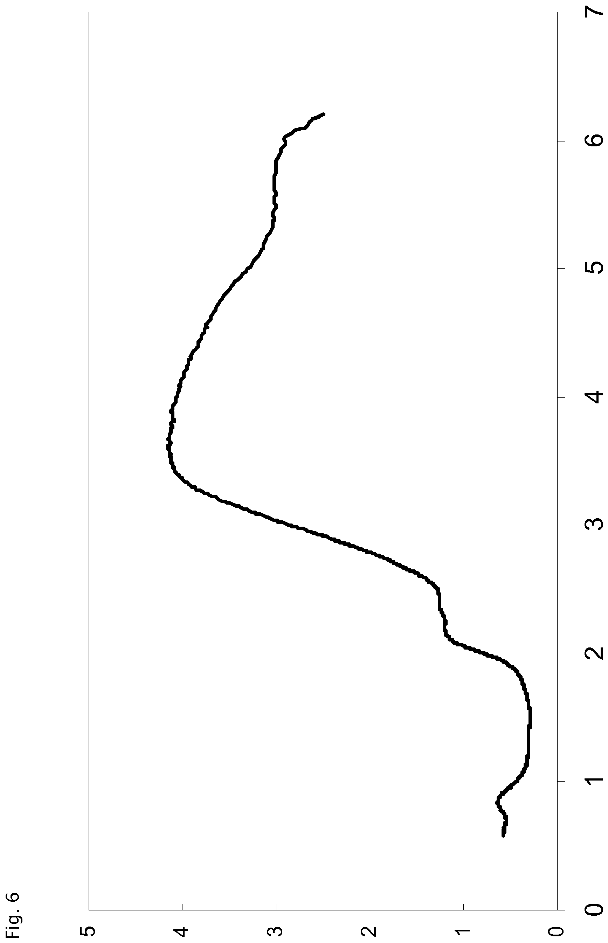

Figur 6 zeigt am Beispiel des Vollkatalysatorformkörpers B3 ein repräsentatives "Pseudo"-absorptionsspektrum im Wellenlängenbereich von 200 nm bis 2126 nm. Auf der Abszisse ist die Kubelka-Munk Absorption, auf der Ordinate die Energie der die Probe bestrahlenden elektromagnetischen Wellen in eV aufgetragen. Der Kubelka-Munk Formalismus (vgl. P. Kubelka, F.Munk, Z.Tech.Phys. 1931, 12, S. 593 ff) wurde genutzt, um die Reflexionsmessung an der Probe in ein entsprechendes Absorptionsspektrum umzuwandeln. Als Messgerät wurde das UV/VIS/NIR-Spektralphotometer Lambda 900 mit einer 150 mm Ulbrichtkugel und als Referenzmittel Spektralon Weiß-Standard der Fa. Labsphere verwendet. Die Messungen erfolgten jeweils an einer Verreibung der jeweiligen Vollkatalysatorformkörper auf eine Partikelgröße < 0,1 mm mit Mörser und Pistill. Die Messparameter bei der UV/VIS/NIR-Spektroskopie waren folgende: Datenintervall: 1nm; Spaltbreite: 2,0 nm/NIR auto; UV/VIS Integrationszeit: 0,44 sec / NIR 0,44 sec; Messgeschwindigkeit: 125 nm/min.

Tabelle 2 zeigt die für die verschiedenen Multimetalloxide der ringförmigen Vollkatalysatorformkörper ermittelten Parameter A (Bedingung 1), a/A (Bedingung 2) und b/c (Bedingung 3).

Tabelle 2

| Vollkatalysatorformkörper | A | a/A | b/c |
|---|---|---|---|
| B1 | 1 | 0,6 | 4,0 |
| B2 | 1 | 0,6 | 4,0 |
| B3 | 0,5 | 1,2 | 4,1 |
| B4 | 0,5 | 1,2 | 4,1 |
| V1 | -0,15 | -4 | 3,8 |
| V2 | 1 | 0,1 | 4,0 |
| V3 | 1 | 2 | 4,0 |
| V4 | 0,5 | 1,2 | 2,3 |
| V5 | 0,5 | 1,2 | 2,3 |
| V6 | 0,5 | 1,2 | 1,0 |
| V7 | 0,5 | 1,2 | 4,0 |

II. Katalyse einer heterogen katalysierten partiellen Gasphasenoxidation von Propen zu Acrolein (Hauptprodukt) und Acrylsäure (Nebenprodukt) mit den in I. erzeugten ringförmigen Vollkatalysatorformkörpern

Ein Reaktionsrohr (V2A Stahl, 12 mm Außendurchmesser, 3 mm Wandstärke, 15 mm Innendurchmesser, 120 cm Länge) wurde von oben nach unten (in der späteren Strömungsrichtung des Reaktionsgasgemischs) wie folgt beschickt:

Abschnitt 1:    30 cm Länge

(fortgesetzt)

40 g Steatitkugeln (Steatit C220 von CeramTec) mit einem Durchmesser von 1,5 bis 2,0 mm als Vorschüttung;

Abschnitt 2: 70 cm Länge

Katalysatorbeschickung mit 100 g des jeweiligen in I. hergestellten ringförmigen Vollkatalysators.

Die Temperierung des Reaktionsrohres erfolgte jeweils mittels einer mit molekularem Stickstoff durchperlten, die Salzbadtemperatur $T^{SB}$ von 320 °C aufweisenden Salzschmelze (53 Gew.-% Kaliumnitrat, 40 Gew.-% Natriumnitrit und 7 Gew.-% Natriumnitrat). Das Salzbad (die Salzschmelze) befand sich in einer zylindrischen Einhüllenden des Innendurchmessers 15 mm. Die zylindrische Einhüllende wies dieselbe Länge wie das Reaktionsrohr auf. Letzteres war in der zylindrischen Einhüllenden so von oben nach unten geführt, dass die beiden Symmetrieachsen zusammenfielen. Der in das Salzbad von unten eingeperlte Stickstoffstrom betrug 40 Nl/h (Normalbedingungen = 1,01 bar, 0 °C). Die Wärmeverluste des Salzbades an die Umgebung waren größer als die vom Rohrreaktor während der Partialoxidation an das Salzbad abgegebene Reaktionswärme. Das Salzbad wurde daher mittels elektrischer Beheizung auf seiner Temperatur $T^{SB}$ (°C) gehalten. Auf diese Weise wurde gewährleistet, dass die Außenwand des Reaktionsrohres stets die entsprechende Temperatur $T^{SB}$ (°C) aufwies.

Das Reaktionsrohr wurde kontinuierlich mit einem Reaktionsgaseingangsgemisch beschickt, das folgende Zusammensetzung aufwies:

5 Vol.-% Propen (des Reinheitsgrades polymer grade)

9,5 Vol.-% molekularer Sauerstoff, und

85,5 Vol.-% molekularer Stickstoff.

Die Stromstärke des Reaktionsgaseingangsgemischstroms wurde jeweils so eingestellt, dass der, bezogen auf einen einmaligen Durchgang des Reaktionsgaseingangsgemischs durch das Reaktionsrohr, resultierende Propenumsatz, bei der Salzbadtemperatur $T^{SB}$ = 320 °C, 95 mol-% betrug. Der Druck am Eingang in das Reaktionsrohr betrug in allen Fällen 1,2 bar absolut (am Reaktionsrohrausgang befand sich zur Eingangsdruckeinstellung ein diesbezügliches Regelventil).

Tabelle 3 zeigt für die verschiedenen Vollkatalysatorformkörper die Propenbelastung PL des Katalysatorfestbetts (hier in Nl Propen/(100 g Vollkatalysatorformkörper · h), die mit der Propenumsatzforderung verträglich war, sowie die nach einer Betriebsdauer von 90 h resultierende Gesamtzielproduktselektivität ($S^{AC+AS}$ (mol-%)) der Gesamtwertproduktbildung an Acrolein (AC) und an Acrylsäure (AS).

Tabelle 3

| Vollkatalysatorformkörper | PL (NL/100 g·h) | $S^{AC+AS}$ (mol-%) |
|---|---|---|
| B1 | 9 | 96,8 |
| B2 | 7 | 96,5 |
| B3 | 10 | 97,0 |
| B4 | 8,5 | 96,5 |
| V1 | 10 | 95,5 |
| V2 | 6 | 96,0 |
| V3 | 10 | 94,9 |
| V4 | 7 | 95,9 |
| V5 | 5,5 | 95,8 |
| V6 | 5 | 95,8 |
| V7 | 6 | 96,1 |

Die in Tabelle 3 ausgewiesenen Ergebnisse zeigen deutlich, dass die beispielhaften (B) Vollkatalysatorformkörper mit erfindungsgemäßer Stöchiometrie ihres Multimetalloxids die bessere Katalysatorperformance sowohl im Hinblick auf die Aktivität (höhere Propenbelastungen sind mit Umsatzziel zuträglich), als auch auf die Gesamtzielproduktselektivität im Vergleich zu den vergleichsbeispielhaften (V) Vollkatalysatorformkörpern aufweisen.

Die bei einer Badtemperatur von 320 °C mit einem Propenumsatzziel von 95 Mol-% noch verträgliche Propenlast PL reflektiert die Aktivität des verwendeten Katalysators. Je höher "PL", desto höher ist die Aktivität. Die in Tabelle 3 berichteten Ergebnisse weisen zusätzlich aus, dass ein erhöhter relativer Wassergehalt beim Vermischen von wässriger Lösung A mit wässriger Lösung B sowohl für die resultierende Aktivität als auch für die resultierende Gesamtzielproduktselektivität förderlich ist (B1 ist besser als B2; B3 ist besser als B4; V4 ist besser als V5; die Multimetalloxide der Vergleichspaare weisen stets eine einheitliche Stöchiometrie auf).

## Patentansprüche

1.  Mo, Bi und Fe enthaltende Multimetalloxidmasse der allgemeinen Stöchiometrie I,

$$Mo_{12} Bi_a Co_b Fe_c K_d Si_e O_x \quad (I),$$

in der die Variablen folgende Bedeutung haben:

a = 0,5 bis 1,
b = 7 bis 8,5,
c = 1,5 bis 3,0,
d = 0 bis 0,15,
e = 0 bis 2,5 und
x = eine Zahl, die durch die Wertigkeit und Häufigkeit der von Sauerstoff verschiedenen Elemente in I bestimmt wird,
und die folgenden Bedingungen erfüllen:

Bedingung 1: $12 - b - 1,5 \cdot c = A$,
und
$0,5 \leq A \leq 1,5$;
Bedingung 2: $0,2 \leq a/A \leq 1,3$; und
Bedingung 3: $2,5 \leq b/c \leq 9$.

2.  Multimetalloxidmasse nach Anspruch 1, deren stöchiometrischer Koeffizient d 0,04 bis 0,1 beträgt.

3.  Multimetalloxidmasse nach Anspruch 1 oder 2, deren stöchiometrischer Koeffizient e 0,5 bis 2 beträgt.

4.  Multimetalloxidmasse nach einem der Ansprüche 1 bis 3, die die Bedingung 1, $0,5 \leq A \leq 1,25$, erfüllt.

5.  Multimetalloxidmasse nach einem der Ansprüche 1 bis 4, die die Bedingung 2, $0,3 \leq a/A \leq 1,2$, erfüllt.

6.  Multimetalloxidmasse nach einem der Ansprüche 1 bis 5, die die Bedingung 3, $3 \leq b/c \leq 9$, erfüllt.

7.  Schalenkatalysator, umfassend einen Trägerformkörper und eine auf der äußeren Oberfläche des Trägerformkörpers befindliche Schale aus wenigstens einer Multimetalloxidmasse gemäß einem der Ansprüche 1 bis 6.

8.  Vollkatalysatorformkörper, dessen Aktivmasse wenigstens ein Multimetalloxid gemäß einem der Ansprüche 1 bis 6 ist.

9.  Vollkatalysatorformkörper nach Anspruch 8, der die Geometrie eines Ringes mit einer Wanddicke von 1 bis 3 mm, einer Länge von 2 bis 10 mm und einem Außendurchmesser von 2 bis 10 mm aufweist.

10. Verfahren zur Herstellung einer Multimetalloxidmasse gemäß eines der Ansprüche 1 bis 6, **dadurch gekennzeichnet, dass** man von Quellen ihrer elementaren Konstituenten ein feinteiliges Trockengemisch erzeugt und dieses bei Temperaturen im Bereich von 350 bis 650°C kalciniert.

11. Verfahren der heterogen katalysierten Gasphasenoxidation eines 3 bis 6 C-Atome aufweisenden Alkans, Alkanols, Alkanals, Alkens und/oder Alkenals an einem Katalysatorbett, **dadurch gekennzeichnet, dass** das Katalysatorbett wenigstens eine Multimetalloxidmasse gemäß einem der Ansprüche 1 bis 6, oder wenigstens einen Katalysator gemäß einem der Ansprüche 7 bis 9, oder wenigstens ein Produkt eines Verfahrens gemäß Anspruch 10 aufweist.

12. Verfahren nach Anspruch 11, **dadurch gekennzeichnet, dass** es ein Verfahren der heterogen katalysierten partiellen Gasphasenoxidation von Propen zu Acrolein ist.

13. Verwendung wenigstens eines Multimetalloxids gemäß eines der Ansprüche 1 bis 6, oder wenigstens eines Katalysators gemäß eines der Ansprüche 7 bis 9, oder wenigstens eines Produkts eines Verfahrens gemäß Anspruch 10 zur Katalyse eines Verfahrens der heterogen katalysierten partiellen Gasphasenoxidation eines 3 bis 6 C-Atome aufweisenden Alkans, Alkanols, Alkanals, Alkens und/oder Alkenals an einem Katalysatorbett.

**Claims**

1. An Mo-, Bi- and Fe-comprising multimetal oxide composition of the general stoichiometry I,

$$Mo_{12} Bi_a Co_b Fe_c K_d Si_e O_x \qquad (I),$$

where the variables have the following meanings:

a = 0.5 to 1,
b = 7 to 8.5,
c = 1.5 to 3.0,
d = 0 to 0.15,
e = 0 to 2.5 and
x = a number which is determined by the valence and abundance of the elements other than oxygen in I and fulfil the following conditions:

condition 1: $12 - b - 1.5 \cdot c = A$,
and
$0.5 \leq A \leq 1.5$;
condition 2: $0.2 \leq a/A \leq 1.3$; and
condition 3: $2.5 \leq b/c \leq 9$.

2. The multimetal oxide composition according to claim 1 whose stoichiometric coefficient d is from 0.04 to 0.1.

3. The multimetal oxide composition according to claim 1 or 2 whose stoichiometric coefficient e is from 0.5 to 2.

4. The multimetal oxide composition according to any of claims 1 to 3 which fulfils the condition 1, $0.5 \leq A \leq 1.25$.

5. The multimetal oxide composition according to any of claims 1 to 4, which fulfils the condition 2, $0.3 \leq a/A \leq 1.2$.

6. The multimetal oxide composition according to any of claims 1 to 5, which fulfils the condition 3, $3 \leq b/c \leq 9$.

7. A coated catalyst comprising a shaped support body and a coating of at least one multimetal oxide composition according to any of claims 1 to 6 present on the outer surface of the shaped support body.

8. An all-active shaped catalyst body whose active composition is at least one multimetal oxide according to any of claims 1 to 6.

9. The all-active shaped catalyst body according to claim 8 which has the geometry of a ring having a wall thickness of from 1 to 3 mm, a length of from 2 to 10 mm and an external diameter of from 2 to 10 mm.

10. A process for preparing a multimetal oxide composition according to any of claims 1 to 6, wherein a finely divided dry mix is produced from sources of elemental constituents of the multimetal oxide composition and this mixture is calcined at temperatures in the range from 350 to 650°C.

11. The process for the heterogeneously catalyzed gas-phase oxidation of an alkane, alkanol, alkanal, alkene and/or alkenal having from 3 to 6 carbon atoms over a catalyst bed, wherein the catalyst bed comprises at least one multimetal oxide composition according to any of claims 1 to 6 or at least one catalyst according to any of claims 7 to 9 or at least one product of a process according to claim 10.

12. The process according to claim 11, wherein it is a process for the heterogeneously catalyzed partial gas-phase oxidation of propene to acrolein.

13. The use of at least one multimetal oxide according to any of claims 1 to 6 or of at least one catalyst according to any of claims 7 to 9 or of at least one product of a process according to claim 10 for the catalysis of a process for the heterogeneously catalyzed partial gas-phase oxidation of an alkane, alkanol, alkanal, alkene and/or alkenal having from 3 to 6 carbon atoms over a catalyst bed.

**Revendications**

1. Masse d'oxyde de plusieurs métaux contenant Mo, Bi et Fe, de la stœchiométrie générale I :

$$Mo_{12} \, Bi_a \, Co_b \, Fe_c \, K_d \, Si_e \, O_x \qquad (I)$$

dans laquelle les variables ont la signification suivante :

a = 0, 5 à 1,
b = 7 à 8, 5,
c = 1,5 à 3, 0,
d = 0 à 0,15,
e = 0 à 2, 5, et
x = un nombre qui est déterminé par la valence et la fréquence des éléments différents de l'oxygène dans I, et remplissent les conditions suivantes :

condition 1 : $12 - b - 1{,}5 \cdot c = A$
et
$0{,}5 \leq A \leq 1{,}5$ ;
condition 2 : $0{,}2 \leq a/A \leq 1{,}3$ ; et
condition 3 : $2{,}5 \leq b/c \leq 9$.

2. Masse d'oxyde de plusieurs métaux selon la revendication 1, dont le coefficient stœchiométrique d est de 0,04 à 0,1.

3. Masse d'oxyde de plusieurs métaux selon la revendication 1 ou 2, dont le coefficient stœchiométrique e est de 0,5 à 2.

4. Masse d'oxyde de plusieurs métaux selon l'une quelconque des revendications 1 à 3, qui remplit la condition 1 : $0{,}5 \leq A \leq 1{,}25$.

5. Masse d'oxyde de plusieurs métaux selon l'une quelconque des revendications 1 à 4, qui remplit la condition 2 : $0{,}3 \leq a/A \leq 1{,}2$.

6. Masse d'oxyde de plusieurs métaux selon l'une quelconque des revendications 1 à 5, qui remplit la condition 3 : $3 \leq b/c \leq 9$.

7. Catalyseur à enveloppe, comprenant un corps moulé support et une enveloppe en au moins une masse d'oxyde de plusieurs métaux selon l'une quelconque des revendications 1 à 6 qui se trouve sur la surface extérieure du corps moulé support.

8. Corps moulé catalytique entier, dont la masse active est au moins un oxyde de plusieurs métaux selon l'une quelconque des revendications 1 à 6.

9. Corps moulé catalytique entier selon la revendication 8, qui présente la géométrie d'un anneau ayant une épaisseur de paroi de 1 à 3 mm, une longueur de 2 à 10 mm et un diamètre extérieur de 2 à 10 mm.

10. Procédé de fabrication d'une masse d'oxyde de plusieurs métaux selon l'une quelconque des revendications 1 à 6, **caractérisé en ce qu'**un mélange sec finement divisé est formé à partir de sources de ses constituants élémentaires, et celui-ci est calciné à des températures dans la plage allant de 350 à 650 °C.

11. Procédé d'oxydation en phase gazeuse sous catalyse hétérogène d'un alcane, alcanol, alcanal, alcène et/ou alcénal comprenant 3 à 6 atomes C sur un lit catalytique, **caractérisé en ce que** le lit catalytique comprend au moins une masse d'oxyde de plusieurs métaux selon l'une quelconque des revendications 1 à 6, ou au moins un catalyseur selon l'une quelconque des revendications 7 à 9, ou au moins un produit d'un procédé selon la revendication 10.

12. Procédé selon la revendication 11, **caractérisé en ce qu'**il consiste en un procédé d'oxydation partielle en phase gazeuse sous catalyse hétérogène de propène en acroléine.

13. Utilisation d'au moins un oxyde de plusieurs métaux selon l'une quelconque des revendications 1 à 6, ou d'au moins un catalyseur selon l'une quelconque des revendications 7 à 9, ou d'au moins un produit d'un procédé selon la revendication 10 pour la catalyse d'un procédé d'oxydation partielle en phase gazeuse sous catalyse hétérogène d'un alcane, alcanol, alcanal, alcène et/ou alcénal comprenant 3 à 6 atomes C sur un lit catalytique.

Fig. 1

Fig. 2

EP 2 731 715 B1

Fig. 3

EP 2 731 715 B1

Fig. 4

Fig. 5

EP 2 731 715 B1

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19855913 A
- DE 10063162 A
- DE 102005037678 A
- DE 10059713 A
- DE 10049873 A
- DE 102007003076 A
- DE 102008054586 A
- DE 102007005606 A
- DE 102007004961 A
- DE 102006044520 A
- DE 2909671 A
- EP 293859 A
- EP 714700 A
- DE 4442346 A
- WO 2008087116 A
- DE 102008042060 A
- WO 2005030393 A
- US 20050131253 A
- WO 2007017431 A
- DE 102008040093 A
- DE 102008040094 A
- WO 02062737 A
- EP 184790 A
- DE 10046957 A
- WO 0224620 A
- WO 200642459 A
- WO 0249757 A
- EP 575897 A
- WO 2007082827 A

- WO 2005113127 A
- WO 2005047224 A
- WO 2005042459 A
- DE 102010048405 A
- DE 102009047291 A
- DE 102008042064 A
- DE 102008042061 A
- EP 990636 A
- EP 1106598 A
- DE 10246119 A
- DE 10245585 A
- DE 4407020 A
- DE 10232748 A
- DE 4431957 A
- EP 700714 A
- EP 700893 A
- EP 468290 B
- DE 19910506 A
- DE 10313213 A
- DE 10313208 A
- DE 19948523 A
- DE 19948248 A
- DE 10313209 A
- WO 0053557 A
- WO 0053558 A
- WO 0136364 A
- EP 873783 A
- DE 10337788 A
- DE 10200804093 A
- WO 2010066645 A
- DE 102009047291 A1
- DE 102007004961 A1

**In der Beschreibung aufgeführte Nicht-Patentliteratur**

- Die Tablette. **W.A.RITSCHEL ; A.BAUER-BRANDI.** Handbuch der Entwicklung, Herstellung und Qualitätssicherung. Edition Verlag Aulendorf, 2002
- Thermogravimetrische Materialfeuchtebestimmung. Die Bibliothek der Technik, vol. 229

- **HORST NAGEL.** Grundlagen und praktische Anwendungen. verlag moderne industrie
- **P. KUBELKA ; F.MUNK.** *Z.Tech.Phys.,* 1931, vol. 12, 593